# EUROPÄISCHE PATENTSCHRIFT

(11) **EP 3 880 651 B1**
(45) Veröffentlichungstag und Bekanntmachung des Hinweises auf die Patenterteilung: **02.07.2025**
(21) Anmeldenummer: 19702362.5
(22) Anmeldetag: 23.01.2019
(51) Int. Cl.: C07C 69/675, A61K 31/22, A61K 31/225, A61K 31/351, C07C 67/08

(54) **VERFAHREN ZUR HERSTELLUNG VON POLYOLBASIERTEN ESTERN VON GEGEBENENFALLS ACYLIERTEN HYDROXYCARBONSÄUREN**
METHOD FOR PRODUCING POLYOL-BASED ESTERS OF OPTIONALLY ACYLATED HYDROXY CARBOXYLIC ACIDS
PROCÉDÉ DE PRÉPARATION D'ESTERS À BASE DE POLYOL D'ACIDES HYDROXYCARBOXYLIQUES ÉVENTUELLEMENT ACYLÉS

(30) Priorität: 17.01.2019 WO PCT/EP2019/051127
(43) Veröffentlichungstag der Anmeldung: 22.09.2021
(73) Patentinhaber: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Strehlke, Ingo Kurt
(86) Internationale Anmeldenummer: PCT/EP2019/051547
(87) Internationale Veröffentlichungsnummer: WO 2020/147983

(56) Entgegenhaltungen:
- WO-A1-2013/150153
- WO-A1-95/09145

## Beschreibung

Die vorliegende Erfindung betrifft das Gebiet der Ketokörper und des damit zusammenhängenden Stoffwechsels sowie die Therapie von damit im Zusammenhang stehenden Erkrankungen.

Insbesondere betrifft die vorliegende Erfindung ein Verfahren zur Herstellung von Polyolestern von gegebenenfalls acylierter 3-Hydroxybuttersäure, insbesondere Polyglycerinestern von gegebenenfalls acylierter 3-Hydroxybuttersäure, sowie die auf diese Weise erhältlichen bzw. hergestellten Reaktionsprodukte (d. h. Polyolester von gegebenenfalls acylierter 3-Hydroxybuttersäure, insbesondere Polyglycerinester von gegebenenfalls acylierter 3-Hydroxybuttersäure) und deren Verwendung, insbesondere in pharmazeutischen Zusammensetzungen, wie Arzneimitteln oder Medikamenten, oder in Nahrungsmittel- und/oder Lebensmittelerzeugnissen, sowie deren weitere Anwendungen bzw. Verwendungen.

Des Weiteren betrifft die vorliegende Erfindung pharmazeutische Zusammensetzungen, insbesondere Arzneimittel oder Medikamente, welche die nach dem erfindungsgemäßen Herstellungsverfahren erhältlichen bzw. hergestellten Reaktionsprodukte (d. h. Polyolester von gegebenenfalls acylierter 3-Hydroxybuttersäure, insbesondere Polyglycerinester von gegebenenfalls acylierter 3-Hydroxybuttersäure) umfassen, sowie deren Anwendungen bzw. Verwendungen.

Schließlich betrifft die vorliegende Erfindung Nahrungsmittel- und/oder Lebensmittelerzeugnisse, insbesondere Nahrungsergänzungsmittel, funktionelle Lebensmittel (*Functional Food*)*, Novel Food,* Lebensmittelzusatzstoffe, Nahrungszusätze, diätetische Lebensmittel, Power-Snacks, Appetitzügler und Kraft- und/oder Ausdauersport-Supplements, welche die nach dem erfindungsgemäßen Herstellungsverfahren erhältlichen bzw. hergestellten Reaktionsprodukte (d. h. Polyolester, insbesondere Polyglycerinester, von gegebenenfalls acylierter 3-Hydroxybuttersäure) umfassen, sowie deren Anwendungen bzw. Verwendungen.

Der Begriff der gegebenenfalls acylierten 3-Hydroxybuttersäure, wie er erfindungsgemäß verwendet wird, bezeichnet insbesondere eine 3-Hydroxybuttersäure, deren 3-Hydroxylfunktion gegebenenfalls acyliert ist, insbesondere acetyliert oder propionyliert ist, vorzugsweise acetyliert ist (d. h. das Wasserstoffatom der 3-Hydroxylfunktion der 3-Hydroxybuttersäure ist gegebenenfalls durch eine Acylgruppe, d. h. eine Gruppe - C(O) - R mit R = Alkyl ersetzt, insbesondere durch eine Acetylgruppe - C(O) - CH₃ oder Propionylgruppe - C(O) - C₂H₅, vorzugsweise durch eine Acetylgruppe - C(O) - CH₃); Entsprechendes gilt folglich auch für das betreffende Carbonsäureanhydrid der 3-Hydroxybuttersäure, d. h. dessen acylierte Form. Folglich weisen die erfindungsgemäßen Reaktionsprodukte (d. h. Polyolester von gegebenenfalls acylierter 3-Hydroxybuttersäure, insbesondere Polyglycerinester von gegebenenfalls acylierter 3-Hydroxybuttersäure) gegebenenfalls die betreffende Acylierung in der 3-Position des 3-Hydroxybuttersäurerests bzw. -teils im betreffenden Ester auf; mit anderen Worten ist beispielsweise ein Polyolester von acylierter 3-Hydroxybuttersäure synonym bzw. gleichbedeutend zu einem Polyolester von 3-Acyloxybuttersäure etc. (Beispielsweise ist ein Polyolester von acetylierter 3-Hydroxybuttersäure synonym bzw. gleichbedeutend zu einem Polyolester von 3-Acetoxybuttersäure etc.).

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glycogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als Ketonkörper bezeichnet oder Englisch auch als *"Keton Bodies"* bezeichnet).

Der Begriff der Ketokörper ist insbesondere eine Sammelbezeichnung für drei Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter den Begriff der Ketokörper fasst man insbesondere die drei Verbindungen Acetoacetat (synonym auch Acetacetat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen, wobei letztere Verbindung die bedeutendste der drei vorgenannten Verbindungen ist. 3-Hydroxybuttersäure bzw. deren Salz kommt physiologisch als (R)-Enantiomer vor, d. h. als (R)-3-Hydroxybuttersäure (synonym auch (3R)-3-Hydroxybuttersäure genannt, um das Chiralitätszentrum in 3-Position hervorzuheben) bzw. deren Salz.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschliche Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitochondrien der Leber statt (Lynenzyklus), wobei 3-Hydroxybutyrat schließlich im Zytosol durch die D-beta-Hydroxybutyrat-Dehydrogenase entsteht. HMG-CoA ist außerdem ein Endprodukt beim Abbau der Aminosäure Leucin, während Acetoacetat beim Abbau der Aminosäuren Phenylalanin und Tyrosin entsteht.

Durch spontane Decarboxylierung entsteht aus Acetoacetat Aceton; es ist gelegentlich im Atem von Diabetikern und Diäthaltenden wahrzunehmen. Es kann vom Körper nicht weiterverwendet werden. Der Anteil von Aceton an den Ketokörpern ist allerdings gering.

Acetoacetat wird also reduktiv in die physiologisch relevante Form der 3-Hydroxybuttersäure bzw. des 3-Hydroxybutyrats überführt, kann aber auch unter Kohlenstoffdioxidfreisetzung in das physiologisch unbrauchbare Aceton zerfallen, was bei einer schweren Ketose, einer Ketoacidose (z. B. bei Diabetes mellitus Typ 1-Patienten ohne Insulinsubstitution), im Urin und in der Ausatemluft nachweisbar und olfaktorisch wahrnehmbar ist.

3-Hydroxybuttersäure wird derzeit im Bereich des Kraftsports als Natrium-, Magnesium- oder Calcium-Salz eingesetzt und in den Handel gebracht.

Jedoch ist 3-Hydroxybuttersäure für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, C₈- und C₁₀-Fettsäuren) aus den korrespondierenden Triglyceriden beabsichtigt.

Grundsätzlich stellt aber aus pharmazeutischer und klinischer Hinsicht 3-Hydroxybuttersäure demgegenüber ein wirksameres pharmazeutisch-pharmakologisches Zielmolekül, welches nach den Erkenntnissen des Standes der Technik prinzipiell für die Therapie einer Vielzahl von Erkrankungen zum Einsatz kommen könnte, aber aufgrund seiner mangelnden physiologischen Kompatibilität dort nicht zum Einsatz kommen kann (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.).

Die nachfolgende Tabelle veranschaulicht rein beispielhaft, aber keinesfalls beschränkend potentielle Therapiemöglichkeiten bzw. mögliche Indikationen für den Wirkstoff 3-Hydroxybuttersäure.

| **Indikation** | **Therapeutischer Effekt** |
|---|---|
| Schädel-Hirn-Trauma | Unter BHB sinkt die Apoptose und Nekrose-Rate von Nervenzellen. |
| Schlaganfall | Unter BHB sinkt die Apoptose und Nekrose-Rate von Nervenzellen. |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). BHB kann hier als Therapeutikum zum schnelleren Erlangen einer normalen Nahrungsaufnahme eingesetzt werden. |
| Appetitzügler | BHB unterdrückt im Zentralnervensystem (ZNS) das Hungergefühl. |
| Epilepsie | Herkömmliche ketogene Diät zur signifikanten Reduzierung der Häufigkeit von Krampfanfällen hat extrem schlechte Patienten-Verträglichkeit. BHB bietet hier eine unmittelbar wirksame Alternative. |
| Morbus Alzheimer, Demenz | Unter BHB zeigen Patienten eine bessere kognitive Leistung. BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu 3-Hydroxybuttersäure oder deren Salzen ermöglichen, insbesondere im physiologischen Stoffwechsel des menschlichen oder tierischen Körpers.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Die WO 2013/150153 A1 betrifft Ketokörper und Ketokörperester zur oralen Verabreichung zur Verbesserung oder zum Erhalt der Muskelkraft, wobei bestimmte Ester von Hydroxybutyratmonomeren organoleptisch akzeptabel sind und eine hohe Aufnahme vom Darm ins Blut aufweisen sollen, wodurch ein schneller Anstieg der Hydroxybutyratkonzentration im Blut und eine physiologische Reaktion einschließlich einer verbesserten Leistungsabgabe während des Trainings ermöglicht werden soll, sowie Zusammensetzungen, welche diese Ketokörper oder Ketokörperester enthalten.

Die WO 95/09145 A1 betrifft Zusammensetzungen, welche als Nährstoffe nützlich sein sollen, wobei die Zusammensetzungen vorzugsweise wasserlösliche parenterale Nährstoffe sind und Glycerinester von β-Acyloxybutyraten darstellen, wobei die Zusammensetzungen als Ersatz für Glukose bei der intravenösen Nahrungszufuhr nützlich sein sollen.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von 3-Hydroxybuttersäure (d. h. beta-Hydroxybuttersäure bzw. BHB bzw. 3-BHB) oder deren Salzen.

Ein solches Verfahren soll insbesondere die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass Polyolester, insbesondere Polyglycerinester, von gegebenenfalls acylierter 3-Hydroxybuttersäure (beta-Hydroxybuttersäure, BHB bzw. 3-BHB) einen effizienten und physiologisch wirksamen bzw. physiologisch kompatiblen Präkursor und/oder Metaboliten für den Ketokörper 3-Hydroxybuttersäure bzw. deren Salze darstellen, und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher - gemäß einem **ersten** Aspekt der vorliegenden Erfindung - ein Verfahren zur Herstellung von Polyolestern, insbesondere Polyglycerinestern, von gegebenenfalls acylierter 3-Hydroxybuttersäure (beta-Hydroxybuttersäure, BHB bzw. 3-BHB) gemäß Anspruch 1 vor; weitere, insbesondere besondere und/oder vorteilhafte Ausgestaltungen des erfindungsgemäßen Verfahrens sind Gegenstand der diesbezüglichen Verfahrensunteransprüche.

Weiterhin betrifft die vorliegende Erfindung - gemäß einem **zweiten** Aspekt der vorliegenden Erfindung -ein nach dem erfindungsgemäßen Verfahren erhältliches Reaktionsprodukt bzw. einen Polyolester, insbesondere Polyglycerinester, von gegebenenfalls acylierter 3-Hydroxybuttersäure (beta-Hydroxybuttersäure, BHB bzw. 3-BHB) gemäß den diesbezüglichen Ansprüchen (Ansprüche 7 bis 10) bzw. ein diesbezüglich erhältliches Gemisch von mindestens zwei, insbesondere mindestens drei Polyolestern, insbesondere Polyglycerinestern, von gegebenenfalls acylierter 3-Hydroxybuttersäure (beta-Hydroxybuttersäure, BHB bzw. 3-BHB), gemäß dem diesbezüglichen Anspruch (Anspruch 11); weitere, insbesondere besondere und/oder vorteilhafte Ausgestaltungen dieses Erfindungsaspekts sind Gegenstand der diesbezüglichen Unteransprüche.

Gleichermaßen betrifft die vorliegende Erfindung - gemäß einem **dritten** Aspekt der vorliegenden Erfindung - eine pharmazeutische Zusammensetzung, insbesondere Arzneimittel oder Medikament, gemäß dem diesbezüglichen unabhängigen Anspruch (Anspruch 12); weitere, insbesondere besondere und/oder vorteilhafte Ausgestaltungen dieses Erfindungsaspekts sind Gegenstand des diesbezüglichen Unteranspruchs.

Darüber hinaus betrifft die vorliegende Erfindung - gemäß einem **vierten** Aspekt der vorliegenden Erfindung - ein erfindungsgemäßes Reaktionsprodukt bzw. einen erfindungsgemäßen Polyolester, insbesondere Polyglycerinester, von gegebenenfalls acylierter 3-Hydroxybuttersäure (beta-Hydroxybuttersäure, BHB bzw. 3-BHB) bzw. ein erfindungsgemäßes Gemisch von mindestens zwei, insbesondere mindestens drei Polyolestern, insbesondere Polyglycerinestern, von gegebenenfalls acylierter 3-Hydroxybuttersäure (beta-Hydroxybuttersäure, BHB bzw. 3-BHB) zur prophylaktischen und/oder therapeutischen Behandlung bzw. zur Verwendung bei der prophylaktischen und/oder therapeutischen Behandlung von Erkrankungen des menschlichen oder tierischen Körpers gemäß dem diesbezüglichen unabhängigen Anspruch (Anspruch 14).

Des Weiteren betrifft die vorliegende Erfindung - gemäß einem **fünften** Aspekt der vorliegenden Erfindung - die Verwendung eines erfindungsgemäßen Polyolesters, insbesondere Polyglycerinesters, von gegebenenfalls acylierter 3-Hydroxybuttersäure (beta-Hydroxybuttersäure, BHB bzw. 3-BHB) bzw. eines erfindungsgemäßen Gemischs von mindestens zwei, insbesondere mindestens drei Polyolestern, insbesondere Polyglycerinestern, von gegebenenfalls acylierter 3-Hydroxybuttersäure (beta-Hydroxybuttersäure, BHB bzw. 3-BHB) zur Herstellung eines Arzneimittels zur prophylaktischen und/oder therapeutischen Behandlung von Erkrankungen des menschlichen oder tierischen Körpers gemäß dem diesbezüglichen unabhängigen Anspruch (Anspruch 15).

Darüber hinaus betrifft die vorliegende Erfindung - gemäß einem **sechsten** Aspekt der vorliegenden Erfindung - die Verwendung eines erfindungsgemäßen Polyolesters, insbesondere Polyglycerinesters, von gegebenenfalls acylierter 3-Hydroxybuttersäure (beta-Hydroxybuttersäure, BHB bzw. 3-BHB) bzw. eines erfindungsgemäßen Gemischs von mindestens zwei, insbesondere mindestens drei Polyolestern, insbesondere Polyglycerinestern, von gegebenenfalls acylierter 3-Hydroxybuttersäure (beta-Hydroxybuttersäure, BHB bzw. 3-BHB) zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung gemäß dem diesbezüglichen unabhängigen Anspruch (Anspruch 16).

Des Weiteren betrifft die vorliegende Erfindung - gemäß einem **siebten** Aspekt der vorliegenden Erfindung - ein Nahrungsmittel und/oder Lebensmittelerzeugnis gemäß dem diesbezüglichen unabhängigen Anspruch (Anspruch 17).

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Des Weiteren versteht es sich von selbst, dass einzelne Aspekte und Ausführungsformen der vorliegenden Erfindung auch in beliebiger Kombination mit anderen Aspekten und Ausführungsformen der vorliegenden Erfindung als offenbart gelten und insbesondere auch eine beliebige Kombination von Merkmalen und Ausführungsformen, wie sie sich aus den Rückbezügen aller Patentansprüche ergibt, umfangreich als offenbart gilt, und zwar im Hinblick auf alle sich ergebenden Kombinationsmöglichkeiten.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Bereichsangaben erforderlichenfalls abweichen kann, ohne dass er den Rahmen der vorliegenden Erfindung verlässt. Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit ein Verfahren zur Herstellung von Polyolestern, insbesondere Polyglycerinestern, von gegebenenfalls acylierter 3-Hydroxybuttersäure (beta-Hydroxybuttersäure, BHB bzw. 3-BHB),
wobei mindestens eine Verbindung der allgemeinen Formel (I)

   CH₃-CH(OR¹)-CH₂-C(O)-O-C(O)-CH₂-CH(OR¹)-CH₃ (I)
wobei in der allgemeinen Formel (I) der Rest R¹ eine Acylgruppe, ausgewählt aus - C(O) - CH₃ (Acetylgruppe) oder - C(O) - C₂H₅ (Propionylgruppe), bevorzugt - C(O) - CH₃ (Acetylgruppe), darstellt,
mit mindestens einem mindestens zwei Hydroxylgruppen (OH-Gruppen) enthaltenden Polyol (II), insbesondere Polyglycerin, umgesetzt wird, gegebenenfalls gefolgt von einer Hydrolyse der Acylgruppen,
so dass als Reaktionsprodukt (III) ein oder mehrere Polyolester, insbesondere Polyglycerinester, von gegebenenfalls acylierter 3-Hydroxybuttersäure erhalten werden.

Wie zuvor ausgeführt, hat die Anmelderin nämlich vollkommen überraschend herausgefunden, dass die auf diese Weise hergestellten Polyolester, insbesondere Polyglycerinester, von gegebenenfalls acylierter 3-Hydroxybuttersäure (beta-Hydroxybuttersäure, BHB bzw. 3-BHB) effiziente, da physiologisch verträgliche Präkursoren und/oder Metabolite der 3-Hydroxybuttersäure bzw. deren Salzen darstellen, welche pharmazeutisch bzw. klinisch auch in größeren Mengen zum Einsatz kommen können, da sie physiologisch kompatibel sind.

Die vorgenannten Polyolester, insbesondere Polyglycerinester, von gegebenenfalls acylierter 3-Hydroxybuttersäure, welche durch das erfindungsgemäße Herstellungsverfahren erstmals in effizienter Weise zugänglich sind, stellen eine physiologisch und pharmakologisch relevante Alternative zu der freien 3-Hydroxybuttersäure bzw. deren Salzen dar.

Die Herstellung von Polyolestern, insbesondere Polyglycerinestern, von gegebenenfalls acylierter 3-Hydroxybuttersäure mittels herkömmlicher organischer Synthese ist komplex und aufwendig, da 3-Hydroxybuttersäure verstärkt zur Polymerisation und anderen unerwünschten Nebenreaktionen (z. B. Wasserabspaltung, Zersetzung etc.) neigt. Im Rahmen der vorliegenden Erfindung konnte erstmals ein effizient arbeitendes Herstellungsverfahren bereitgestellt werden, mit welchem sich Polyolester, insbesondere Polyglycerinester, von gegebenenfalls acylierter 3-Hydroxybuttersäure ohne unerwünschte Nebenreaktionen herstellen lassen, insbesondere einstufig.

Das erfindungsgemäße Verfahren ermöglicht somit erstmalig die Bereitstellung untoxischer Polyolester, insbesondere Polyglycerinester, von gegebenenfalls acylierter 3-Hydroxybuttersäure aus an sich bekannten, kommerziell verfügbaren und vor allem physiologisch unbedenklichen Komponenten bzw. Edukten (Ausgangsverbindungen). Die resultierenden Polyolester, insbesondere Polyglycerinester, von gegebenenfalls acylierter 3-Hydroxybuttersäure können physiologisch, insbesondere im Magen und/oder im Darm, aufgespalten werden und das Zielmolekül "3-Hydroxybuttersäure" bzw. deren Salze als Wirkstoff bzw. Wirkkomponente freisetzen bzw. generieren.

Darüber hinaus weisen die vorgenannten Polyolester, insbesondere Polyglycerinester, von gegebenenfalls acylierter 3-Hydroxybuttersäure auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Gleichermaßen ermöglicht es das erfindungsgemäße Herstellungsverfahren die Polyolester, insbesondere Polyglycerinester, von gegebenenfalls acylierter 3-Hydroxybuttersäure frei von toxischen Verunreinigungen bereitzustellen.

Darüber hinaus kann bei entsprechenden Ausgangsmaterialien die Herstellung auch enantioselektiv durchgeführt werden. So ermöglicht es beispielsweise das erfindungsgemäße Herstellungsverfahren, die biologisch relevante Form, d. h. das (R)-Enantiomer anzureichern, um bei oraler Verabreichung das renale System von Patienten nicht zu belasten (d. h. Elimination über die Nieren). Grundsätzlich ist es aber auch möglich und kann es unter bestimmten Voraussetzungen zweckdienlich sein, das (S)-Enantiomer anzureichern.

Darüber hinaus ist das erfindungsgemäße Herstellungsverfahren, einschließlich optionaler Weiterverarbeitungs- bzw. Aufreinigungsverfahrensschritte, wirtschaftlich bzw. ökonomisch betreibbar und auch großtechnisch umsetzbar.

Insbesondere verwendet das erfindungsgemäße Herstellungsverfahren kommerziell verfügbare Edukte und ermöglicht darüber hinaus eine relativ einfache Verfahrensführung auch bei großtechnischer Umsetzung.

Im Gegensatz zu herkömmlichen Herstellungsverfahren des Standes der Technik kommt das erfindungsgemäße Herstellungsverfahren ohne komplexe Edukte aus und verläuft nur einstufig. Dennoch werden im Rahmen des erfindungsgemäßen Herstellungsverfahrens exzellente Ausbeuten erzielt, wobei in gleicher Weise die Bildung von Nebenprodukten minimiert bzw. vermieden wird.

Darüber hinaus ist das erfindungsgemäßen Verfahren einfach und wirtschaftlich. Insbesondere wird üblicherweise das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*); folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Das erfindungsgemäße Herstellungsverfahren führt üblicherweise zu einem Gemisch verschiedener Polyolester, insbesondere Polyglycerinester, von gegebenenfalls acylierter 3-Hydroxybuttersäure, d. h. zu einem Gemisch von mindestens zwei, insbesondere mindestens drei voneinander verschiedenen Polyolestern, insbesondere Polyglycerinestern, von gegebenenfalls acylierter 3-Hydroxybuttersäure. Das resultierende Rohreaktionsprodukt bzw. Rohgemisch kann mit an sich bekannten Methoden aufgereinigt werden, insbesondere von gegebenenfalls noch vorhandenen Edukten und/oder gegebenenfalls vorhandenen Nebenprodukten befreit werden, und darüber hinaus - sofern gewünscht - mit ebenfalls an sich bekannten Methoden aufgespalten werden, insbesondere destillativ und/oder chromatographisch (z. B. Fraktionierung in die einzelnen Polyolester, d. h. Mono-, Di-, Tri- usw. Polyolester von gegebenenfalls acylierter 3-Hydroxybuttersäure, oder aber Fraktionierung in Fraktionen mit angereichertem und abgereichertem Anteil einzelner Polyester etc.).

Gemäß einer besonderen Ausführungsform der vorliegenden Erfindung kann die Verbindung der allgemeinen Formel (I) entweder in racemischer Form oder aber in Form des (R)-Enantiomers eingesetzt werden. Die (R)-Konfiguration bezieht sich auf die beiden chiralen Kohlenstoffatome der Verbindung der allgemeinen Formel (I), d. h. die nachfolgend mit "*" gekennzeichneten Kohlenstoffatome in der Verbindung der allgemeinen Formel (I), wobei diese chiralen Zentren jeweils dem C-Atom in 3-Position der 3-Hydroxybuttersäure entsprechen:

CH₃-C*H(OR¹)-CH₂-C(O)-O-C(O)-CH₂-C*H(OR¹)-CH₃ (I).

Erfindungsgemäß bevorzugt ist es, wenn in der allgemeinen Formel (I) der Rest R¹ eine Gruppe - C(O) - CH₃ (Acetylgruppe) darstellt.

Mit anderen Worten ist es erfindungsgemäß bevorzugt, dass als Verbindung der allgemeinen Formel (I) die Verbindung der Formel CH₃- CH(OAc) - CH₂ - C(O) - O - C(O) - CH₂ - CH(OAc) - CH₃ , wobei der Rest Ac eine Acetylgruppe darstellt, eingesetzt wird.

Dies ermöglicht eine besonders effiziente Verfahrensführung und hohe Ausbeuten mit minimierter bzw. unterdrückter Nebenproduktbildung. Zudem ist die Ausgangsverbindung der allgemeinen Formel (I) ohne Weiteres aus herkömmlichen, kommerziell verfügbaren Edukten herzustellen und zudem ökonomisch effizienter als die freie Säure (d. h. 3-Hydroxybuttersäure) umsetzbar.

Insbesondere wird bei dem erfindungsgemäßen Verfahren die Umsetzung in Abwesenheit von Lösemitteln und/oder ohne jedwedes Lösemittel durchgeführt. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß einer besonderen Ausführungsform der vorliegenden Erfindung kann die Umsetzung autokatalytisch oder in Gegenwart eines Katalysators, insbesondere einer mineralischen Säure, durchgeführt werden, vorzugsweise autokatalytisch. Bei dieser besonderen Ausführungsform ist es bevorzugt, wenn die Umsetzung autokatalytisch durchgeführt wird (d. h. in Abwesenheit eines zusätzlichen Katalysators).

Im Rahmen des erfindungsgemäßen Verfahrens können bei der Umsetzung die Temperaturen in weiten Bereichen variieren. Insbesondere kann die Umsetzung bei Temperaturen im Bereich von 50 °C bis 140 °C, vorzugsweise im Bereich von 60 °C bis 130 °C, besonders bevorzugt im Bereich von 70 °C bis 125 °C, ganz besonders bevorzugt im Bereich von 75 °C bis 110 °C, durchgeführt werden. Dennoch kann es anwendungsbezogen oder einzelfallbedingt gegebenenfalls erforderlich werden, von den vorgenannten Werten abzuweichen, ohne dass der Rahmen der vorliegenden Erfindung verlassen ist.

Im Rahmen des erfindungsgemäßen Verfahrens können bei der Umsetzung auch die Drücke in weiten Bereichen variieren. Insbesondere kann die Umsetzung bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden. Dennoch kann es anwendungsbezogen oder einzelfallbedingt gegebenenfalls erforderlich werden, von den vorgenannten Werten abzuweichen, ohne dass der Rahmen der vorliegenden Erfindung verlassen ist.

Was die Menge an Edukten bzw. Ausgangsverbindungen anbelangt, so kann auch diese in weiten Bereichen variiert werden.

Unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (I), bezogen auf die Hydroxylgruppen des Polyols (II), insbesondere Polyglycerins, in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird.

Gleichermaßen unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (I) und das Polyol (II), insbesondere Polyglycerin, in einem Molverhältnis von Verbindung der allgemeinen Formel (I) / Polyol (II) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

Was das im Rahmen des erfindungsgemäßen Verfahrens einsetzbare Polyol (II) anbelangt, so ist es insbesondere bevorzugt, wenn das Polyol (II) mindestens drei Hydroxylgruppen (OH-Gruppen) enthält.

Gemäß einer besonderen Ausführungsform des erfindungsgemäßen Verfahrens kann es insbesondere vorgesehen sein, wenn das im Rahmen des erfindungsgemäßen Verfahrens einsetzbare Polyol (II) der allgemeinen Formel (IIa)

(HO)ₘ-(X)-(OH)ₙ (IIa)

entspricht, wobei in der allgemeinen Formel (IIa)
- X einen organischen Rest, insbesondere einen 4 bis 20 Kohlenstoffatome enthaltenden und gegebenenfalls 1 bis 9 Sauerstoffatome enthaltenden, vorzugsweise gesättigten organischen Rest darstellt, vorzugsweise ausgewählt aus einem Alkylrest oder einem (Poly-)Alkylether-Rest, insbesondere (Poly-)-Alkylenglykolrest, besonders bevorzugt ausgewählt aus einem C₄-C₂₀-Alkylrest oder einem C₄-C₂₀-(Poly-)Alkylether-Rest, insbesondere C₄-C₂₀-(Poly-)Alkylen-glykolrest; und
- die Variablen m und n, jeweils unabhängig voneinander, eine ganze Zahl von 1 bis 10 darstellen.

Insbesondere ist es in diesem Zusammenhang erfindungsgemäß bevorzugt, dass die Hydroxylgruppen des Polyols (II) in beliebigen Positionen des Rests X befindlich sein können, vorzugsweise wobei mindestens eine Hydroxylgruppe endständig ist (d. h. eine primäre Hydroxylgruppe ist). Dies bedeutet insbesondere, dass die Hydroxylgruppen an beliebigen Positionen des organischen Rests X angeordnet bzw. vorgesehen sein können (vorzugsweise jedoch mit der Maßgabe, dass mindestens eine Hydroxylgruppe endständig ist und/oder eine primäre Hydroxylgruppe ist).

Insbesondere kann das im Rahmen des erfindungsgemäßen Verfahrens einsetzbare Polyol (II) ausgewählt sein aus Polyetherpolyolen und Alkanpolyolen sowie deren Kombinationen, insbesondere C₄-C₂₀-Polyetherpolyolen und C₄-C₂₀-Alkanpolyolen, vorzugsweise C₄-C₂₀-Polyetherpolyolen und C₄-C₂₀-Alkandiolen, besonders bevorzugt Polyetherpolyolen, ganz besonders bevorzugt C₄-C₂₀-Polyetherpolyolen.

Gemäß einer besonderen Ausführungsform des erfindungsgemäßen Verfahrens kann das Polyol (II) insbesondere ausgewählt sein aus Polyetherpolyolen, insbesondere C₄-C₂₀-Polyetherpolyolen, vorzugsweise Polyglycerinen der allgemeinen Formel (IIb)

HO-CH₂-CH(OH)-CH₂-[O-CH₂-CH(OH)-CH₂]ₚ-OH (IIb)

wobei in der allgemeinen Formel (IIb) die Variable p eine ganze Zahl von 1 bis 4, insbesondere 1 oder 2, vorzugsweise 1, darstellt.

Gemäß einer weiteren besonderen Ausführungsform des erfindungsgemäßen Verfahrens kann das Polyol (II) ein Diglycerin der Formel (IIc)

HO-CH₂-CH(OH)-CH₂-O-CH₂-CH(OH)-CH₂-OH (IIc)

sein.

Gemäß einer wiederum weiteren besonderen Ausführungsform des erfindungsgemäßen Verfahrens kann das Polyol (II) ausgewählt sein aus Alkandiolen, insbesondere C₄-C₂₀-Alkandiolen, vorzugsweise linearen oder verzweigten Alkandiolen, bevorzugt linearen oder verzweigten C₄-C₂₀-Alkandiolen, besonders bevorzugt linearen C₄-C₂₀-Alkandiolen, ganz besonders bevorzugt linearen C₄-C₂₀-Alkandiolen mit mindestens einer endständigen und/oder primären Hydroxylgruppe, noch mehr bevorzugt Pentandiol, insbesondere 1,2-Pentandiol.

Gemäß einer bevorzugten Ausführungsform der vorliegenden Erfindung betrifft die vorliegende Erfindung gemäß diesem Erfindungsaspekt ein Verfahren zur Herstellung von Polyolestern, insbesondere Polyglycerinestern, von gegebenenfalls acylierter 3-Hydroxybuttersäure (beta-Hydroxybuttersäure, BHB bzw. 3-BHB), insbesondere ein wie zuvor definiertes bzw. beschriebenes Verfahren,
wobei mindestens eine Verbindung der allgemeinen Formel (I)

   CH₃-CH(OR¹)-CH₂-C(O)-O-C(O)-CH₂-CH(OR¹)-CH₃ (I)
wobei in der allgemeinen Formel (I) der Rest R¹ eine Acylgruppe, ausgewählt aus - C(O) - CH₃ (Acetylgruppe) oder - C(O) - C₂H₅ (Propionylgruppe), bevorzugt - C(O) - CH₃ (Acetylgruppe), darstellt,
mit mindestens einem mindestens zwei Hydroxylgruppen (OH-Gruppen) enthaltenden Polyol (II), insbesondere Polyglycerin, umgesetzt wird, wobei das Polyol (II) ausgewählt ist aus Polyetherpolyolen, insbesondere C₄-C₂₀-Polyetherpolyolen, vorzugsweise Polyglycerinen der allgemeinen Formel (IIb)

   HO-CH₂-CH(OH)-CH₂-[O-CH₂-CH(OH)-CH₂]ₚ-OH (IIb)
wobei in der allgemeinen Formel (IIb) die Variable p eine ganze Zahl von 1 bis 4, insbesondere 1 oder 2, vorzugsweise 1, darstellt,
gegebenenfalls gefolgt von einer Hydrolyse der Acylgruppen,
so dass als Reaktionsprodukt (III) ein oder mehrere Polyolester, insbesondere Polyglycerinester, von gegebenenfalls acylierter 3-Hydroxybuttersäure erhalten werden.

Gemäß einer weiteren bevorzugten Ausführungsform der vorliegenden Erfindung betrifft die vorliegende Erfindung gemäß diesem Erfindungsaspekt ein Verfahren zur Herstellung von Polyolestern, insbesondere Polyglycerinestern, von gegebenenfalls acylierter 3-Hydroxybuttersäure (beta-Hydroxybuttersäure, BHB bzw. 3-BHB), insbesondere ein wie zuvor definiertes bzw. beschriebenes Verfahren,
wobei mindestens eine Verbindung der allgemeinen Formel (I)

   CH₃-CH(OR¹)-CH₂-C(O)-O-C(O)-CH₂-CH(OR¹)-CH₃ (I)
wobei in der allgemeinen Formel (I) der Rest R¹ eine Gruppe - C(O) - CH₃ (Acetylgruppe) darstellt,
mit mindestens einem mindestens zwei Hydroxylgruppen (OH-Gruppen) enthaltenden Polyol (II), insbesondere Polyglycerin, umgesetzt wird, wobei das Polyol (II) ausgewählt ist aus Polyglycerinen der allgemeinen Formel (IIb)

   HO-CH₂-CH(OH)-CH₂-[O-CH₂-CH(OH)-CH₂]ₚ-OH (IIb)
wobei in der allgemeinen Formel (IIb) die Variable p eine ganze Zahl 1 oder 2, vorzugsweise 1, darstellt,
gegebenenfalls gefolgt von einer Hydrolyse der Acetylgruppen,
so dass als Reaktionsprodukt (III) ein oder mehrere Polyolester, insbesondere Polyglycerinester, von gegebenenfalls acylierter 3-Hydroxybuttersäure erhalten werden.

Im Rahmen des erfindungsgemäßen Verfahrens wird bei der Umsetzung gleichzeitig die Verbindung gemäß der allgemeinen Formel (IV)

CH₃-CH(OR¹)-CH₂-C(O)-OH (IV)

gebildet wird, wobei in der allgemeinen Formel (IV) der Rest R¹ die zuvor angegebene Bedeutung hat.

Insbesondere ist es in diesem Zusammenhang bevorzugt, wenn die Verbindung gemäß der allgemeinen Formel (IV) nach erfolgter Umsetzung entfernt wird, insbesondere mittels destillativer Entfernung.

Im Rahmen des erfindungsgemäßen Herstellungsverfahrens kann das Reaktionsprodukt (III), insbesondere die Zusammensetzung des Reaktionsprodukts, insbesondere das Vorhandensein verschiedener Polyolester, insbesondere Polyglycerinester, von gegebenenfalls acylierter 3-Hydroxybuttersäure und deren Anteil im Fall eines Gemischs, mittels der Umsetzungsbedingungen kontrolliert und/oder gesteuert werden, insbesondere durch Auswahl der Umsetzungstemperatur (Reaktionstemperatur) und/oder Auswahl des Umsetzungsdrucks (Reaktionsdrucks) und/oder Vorsehen eines Katalysators und dessen Auswahl in Bezug auf Art und/oder Menge und/oder Auswahl der Mengen der Ausgangsverbindungen (Edukte).

Im Anschluss an die Umsetzung kann das erhaltene Reaktionsprodukt weiteren üblichen bzw. an sich bekannten Aufreinigungs- bzw. Aufarbeitungsschritten unterzogen werden.

In diesem Zusammenhang kann das erhaltene Reaktionsprodukt nach erfolgter Umsetzung fraktioniert werden, insbesondere destillativ fraktioniert werden.

Auch können nichtumgesetzte Ausgangsverbindungen (I) und/oder (II) aus dem Reaktionsprodukt abgetrennt und anschließend rezykliert werden.

Wie bereits zuvor im Zusammenhang mit dem erfindungsgemäßen Verfahren erläutert, kann sich im Rahmen des erfindungsgemäßen Verfahrens - gemäß einer besonderen Ausführungsform - der zuvor beschriebenen Umsetzung der mindestens einen Verbindung der allgemeinen Formel (I) mit dem mindestens einem Polyol (II), insbesondere Polyglycerin, optional eine Hydrolyse der Acylgruppen anschließen. Diese Ausführungsform des erfindungsgemäßen Verfahrens ist dann bevorzugt, wenn das Reaktionsprodukt (III) frei von Acylgruppen ausgebildet sein soll. Zu diesem Zweck wird erfindungsgemäß eine selektive bzw. partielle Hydrolyse der nach Umsetzung in den erhaltenen Umsetzungsprodukten vorhandenen Acylgruppen durchgeführt (welche sich in den nach Umsetzung erhaltene Umsetzungsprodukten in 3-Position des 3-Hydroxybuttersäureteils der Umsetzungsprodukte befinden). Auf diese Weise lassen sich also Reaktionsprodukte (III) gemäß nachfolgender Definition (d. h. nachfolgende allgemeine Formeln (IIIa), (IIIb) und (IIIc)) erhalten, bei welchen der Rest R¹ ein Wasserstoffatom darstellt (d. h. Ersetzen der Acylgruppe durch ein Wasserstoffatom im Rahmen der Hydrolyse).

Insbesondere ist es bevorzugt, wenn bei dieser Ausführungsform des erfindungsgemäßen Verfahrens die Hydrolyse der Acylgruppen, insbesondere der Acetylgruppen, in Gegenwart eines Katalysators, vorzugsweise eines Enzyms, erfolgt. Dies gewährleistet eine selektive bzw. partielle Hydrolyse der Acylgruppen, insbesondere unter schonenden und ökonomischen Bedingungen, vorzugsweise unter Vermeidung der Bildung von Nebenprodukten.

Insbesondere kann das für die Hydrolyse der Acylgruppen, insbesondere der Acetylgruppen, eingesetzte Enzym ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstande sind (Lipolyse).

Insbesondere kann sich das für die Hydrolyse der Acylgruppen, insbesondere der Acetylgruppen, eingesetzte Enzym ableiten von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus.*

Insbesondere kann das für die Hydrolyse der Acylgruppen, insbesondere der Acetylgruppen, eingesetzte Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Insbesondere kann das für die Hydrolyse der Acylgruppen, insbesondere der Acetylgruppen, eingesetzte Enzym in Mengen, bezogen auf die Gesamtmenge der zu hydrolysierenden Verbindung, im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden.

Bei dieser besonderen Ausführungsform ist es bevorzugt, wenn das für die Hydrolyse der Acylgruppen, insbesondere der Acetylgruppen, eingesetzte Enzym nach der Hydrolyse rezykliert wird.

Die optional durchgeführte Hydrolyse der Acylgruppen, insbesondere der Acetylgruppen, kann insbesondere bei Temperaturen im Bereich von 10 °C bis 80 °C, insbesondere im Bereich von 20 °C bis 80 °C, vorzugsweise im Bereich von 25°C bis 75 °C, besonders bevorzugt im Bereich von 45 °C bis 75 °C, ganz besonders bevorzugt im Bereich von 50 °C bis 70 °C, durchgeführt werden.

Die optional durchgeführte Hydrolyse der Acylgruppen, insbesondere der Acetylgruppen, kann insbesondere bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Typischerweise wird die Hydrolyse der Acylgruppen, insbesondere der Acetylgruppen, in Gegenwart von Wasser durchgeführt.

Gemäß dieser besonderen Ausführungsform des erfindungsgemäßen Verfahrens, wonach sich der zuvor beschriebenen Umsetzung der mindestens einen Verbindung der allgemeinen Formel (I) mit dem mindestens einem Polyol (II), insbesondere Polyglycerin, eine Hydrolyse der Acylgruppen anschließt, können als zuvor beschriebenes Enzym entsprechende, kommerziell verfügbare Enzyme der vorgenannten Definition verwendet werden (z. B. CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.).

Gemäß einer besonderen Ausführungsform des erfindungsgemäßen Herstellungsverfahrens kann insbesondere derart vorgegangen werden, dass im Reaktionsprodukt nach erfolgter Umsetzung noch vorhandene Hydroxylgruppen zumindest teilweise, vorzugsweise vollständig, funktionalisiert, insbesondere verestert werden. Insbesondere kann sich der Umsetzung eine teilweise, insbesondere vollständige Funktionalisierung, insbesondere Veresterung, noch vorhandener Hydroxylgruppen anschließen.

Bei dieser besonderen Ausführungsform des erfindungsgemäßen Verfahrens kann insbesondere die Funktionalisierung, insbesondere Veresterung, der Hydroxylgruppen durch Reaktion mit einem Carbonsäureanhydrid, insbesondere C₂-C₃₀-Carbonsäureanhydrid, bevorzugt C₂-C₁₀-Carbonsäureanhydrid, vorzugsweise C₇-Carbonsäureanhydrid, erfolgen. Bei dem C₂-C₃₀-Carbonsäureanhydrid, bevorzugt C₂-C₁₀-Carbonsäureanhydrid, kann es sich um ein lineares (d. h. geradkettiges) oder verzweigtes, gesättigtes oder ein- oder mehrfach ungesättigtes C₂-C₃₀-Carbonsäureanhydrid, bevorzugt C₂-C₁₀-Carbonsäureanhydrid, handeln.

Bei einer weiteren besonderen Ausführungsform des erfindungsgemäßen Verfahrens kann insbesondere die Funktionalisierung, insbesondere Veresterung, der Hydroxylgruppen durch Reaktion mit einem Carbonsäureanhydrid, insbesondere mit einer Verbindung der allgemeinen Formel (I), wie zuvor definiert, durchgeführt werden.

Eine erfindungsgemäß besonders bevorzugte Vorgehensweise, welche eine Funktionalisierung, insbesondere Veresterung, noch vorhandener Hydroxylgruppen im Anschluss an die Umsetzung einschließlich erfolgter Hydrolyse der Acylgruppen vorsieht, wird durch das nachfolgende Reaktions- bzw. Syntheseschema mit Diglycerin als Ausgangspolyol veranschaulicht (wobei in Abhängigkeit von der Reaktionsführung bei der Umsetzung entweder einzelne der Ester oder ein Gemisch von zwei oder mehreren hiervon erhalten werden und wobei in dem nachfolgenden Reaktions- bzw. Syntheseschema der Rest Y entweder einen Rest der Formel CH₃ - (CH₂)_{x = 0-28} - C(O) - bezeichnet, sofern die Funktionalisierung mit einem C₂-C₃₀-Carbonsäureanhydrid durchgeführt wird, oder aber einen Rest der Formel CH₃ - CH(OR¹) - CH₂ - C(O) - , wobei darin der Rest R¹ die zuvor angegebene Bedeutung hat, bezeichnet, sofern die Funktionalisierung mit einem Carbonsäureanhydrid entsprechend einer Verbindung der allgemeinen Formel (I), wie zuvor definiert, durchgeführt wird):

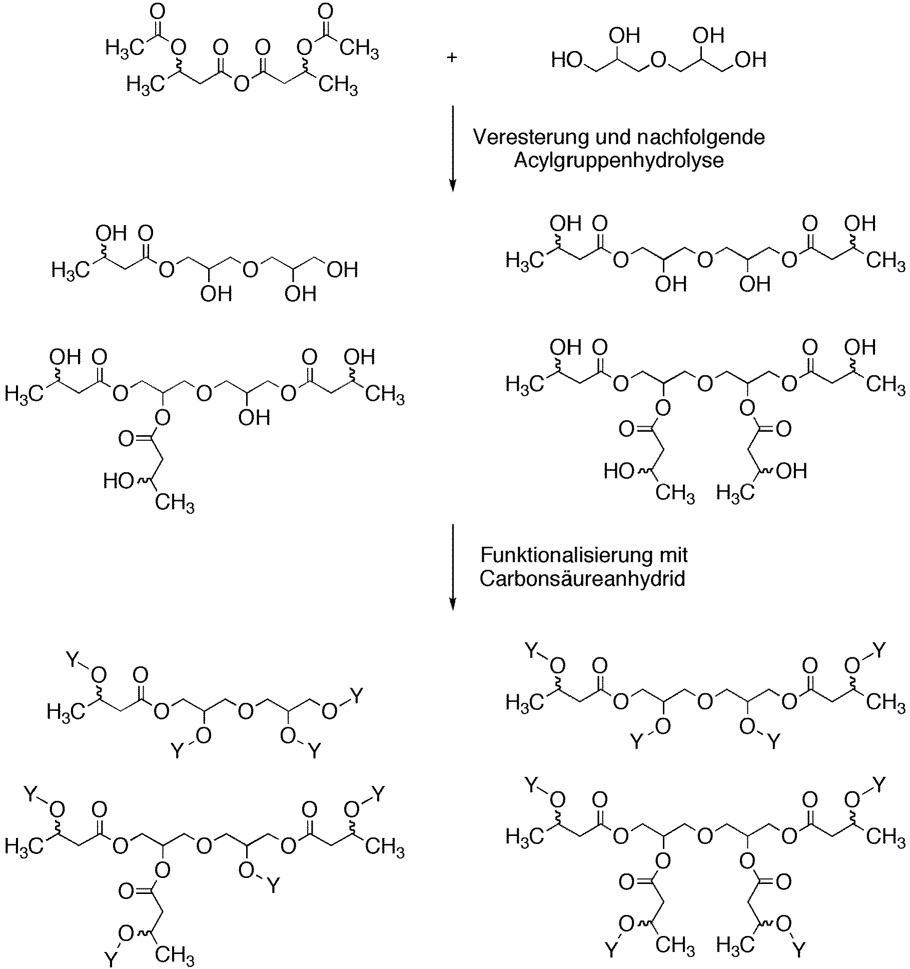

Was die im Rahmen des erfindungsgemäßen Verfahrens eingesetzte Verbindung der allgemeinen Formel (I), wie zuvor definiert, anbelangt, so ist diese erhältlich und/oder wird diese erhalten durch Umsetzung von einem Carbonsäureanhydrid der Formel (V)

R¹ - O - R¹ (V)

wobei der Rest R¹ die zuvor angegebene Bedeutung hat, insbesondere von Essigsäureanhydrid (Acetanhydrid) oder Propionsäureanhydrid, vorzugsweise von Essigsäureanhydrid (Acetanhydrid), mit 3-Hydroxybuttersäure.

Insbesondere kann dabei die Umsetzung von Carbonsäureanhydrid der Formel (V) mit 3-Hydroxybuttersäure entsprechend der Reaktionsgleichung 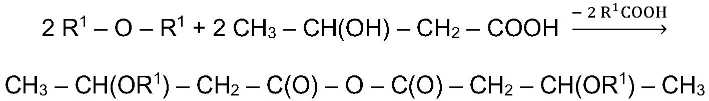 erfolgen, wobei in der Reaktionsgleichung der Rest R¹ die zuvor angegebene Bedeutung hat.

Gemäß einer besonderen Ausführungsform kann die Umsetzung von Essigsäureanhydrid (Acetanhydrid) mit 3-Hydroxybuttersäure entsprechend der Reaktionsgleichung 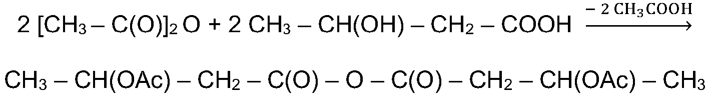 erfolgen, wobei in der Reaktionsgleichung der Rest Ac eine Acetylgruppe darstellt.

Die Temperaturen der Umsetzung von Carbonsäureanhydrid der Formel (V), wie zuvor definiert, mit 3-Hydroxybuttersäure können in weiten Bereichen variieren. Insbesondere kann die Umsetzung von Carbonsäureanhydrid der Formel (V), wie zuvor definiert, mit 3-Hydroxybuttersäure bei Temperaturen im Bereich von 60 bis 150 °C, insbesondere im Bereich von 70 bis 120 °C, vorzugsweise im Bereich von 80 bis 100 °C, durchgeführt werden.

Die Drücke der Umsetzung von Carbonsäureanhydrid der Formel (V), wie zuvor definiert, mit 3-Hydroxybuttersäure können gleichermaßen in weiten Bereichen variieren. Insbesondere kann die Umsetzung von Carbonsäureanhydrid der Formel (V), wie zuvor definiert, mit 3-Hydroxybuttersäure bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Gemäß einer besonderen Ausführungsform des erfindungsgemäßen Verfahrens betrifft die vorliegenden Erfindung ein Verfahren zur Herstellung von Polyolestern, insbesondere Polyglycerinestern, von gegebenenfalls acylierter 3-Hydroxybuttersäure (beta-Hydroxybuttersäure, BHB bzw. 3-BHB), insbesondere ein wie zuvor beschriebenes Verfahren, wobei
(a) in einem ersten Verfahrensschritt (a) ein Carbonsäureanhydrid der zuvor definierten Formel (V)

   R¹ - O - R¹ (V)

   wobei der Rest R¹ die zuvor angegebene Bedeutung hat, insbesondere Essigsäureanhydrid (Acetanhydrid) oder Propionsäureanhydrid, vorzugsweise Essigsäureanhydrid (Acetanhydrid), mit 3-Hydroxybuttersäure umgesetzt wird, so dass eine Verbindung der allgemeinen Formel (I), wie zuvor definiert, erhalten wird; und nachfolgend
(b) in einem zweiten Verfahrensschritt (b) die auf diese Weise erhaltene Verbindung der allgemeinen Formel (I), wie zuvor definiert, mit mindestens einem mindestens zwei Hydroxylgruppen (OH-Gruppen) enthaltenden Polyol (II), insbesondere Polyglycerin, wie zuvor definiert, umgesetzt wird;
(c) gegebenenfalls gefolgt von einem dritten Verfahrensschritt (c) der Hydrolyse der Acylgruppe,
so dass als Reaktionsprodukt (III) ein oder mehrere Polyolester, insbesondere Polyglycerinester, von gegebenenfalls acylierter 3-Hydroxybuttersäure erhalten werden.

Im Rahmen des erfindungsgemäßen Verfahrens lassen sich als Reaktionsprodukt (III) insbesondere ein oder mehrere Polyolester, insbesondere Polyglycerinester, von gegebenenfalls acylierter 3-Hydroxybuttersäure der allgemeinen Formel (IIIa)

(R²O)ₘ - (X) - (OR²)ₙ (IIIa)

erhalten,
wobei in der allgemeinen Formel (IIIa)
- X einen organischen Rest, insbesondere einen 4 bis 20 Kohlenstoffatome enthaltenden und gegebenenfalls 1 bis 9 Sauerstoffatome enthaltenden, vorzugsweise gesättigten organischen Rest darstellt, vorzugsweise ausgewählt aus einem Alkylrest oder einem (Poly-)Alkylether-Rest, insbesondere (Poly-)-Alkylenglykolrest, besonders bevorzugt ausgewählt aus einem C₄-C₂₀-Alkylrest oder einem C₄-C₂₀-(Poly-)Alkylether-Rest, insbesondere C₄-C₂₀-(Poly-)Alkylenglykolrest,
- die Variablen m und n, jeweils unabhängig voneinander, eine ganze Zahl von 1 bis 10 darstellen,
- R², unabhängig voneinander, darstellt: Wasserstoff, einen Rest CH₃ - CH(OH) - CH₂ - C(O) - oder einen Rest CH₃ - CH(OR¹) - CH₂ - C(O) - mit R¹ = Acylgruppe, ausgewählt aus - C(O) - CH₃ (Acetylgruppe) oder - C(O) - C₂H₅ (Propionylgruppe), bevorzugt - C(O) - CH₃ (Acetylgruppe), jedoch mit der Maßgabe, dass mindestens ein Rest R², insbesondere mindestens zwei Reste R², keinen Wasserstoff darstellt, und/oder mit der Maßgabe, dass mindestens ein Rest R², insbesondere mindestens zwei Reste R², einen Rest CH₃-CH(OH) - CH₂ - C(O) - oder einen Rest CH₃- CH(OR¹) - CH₂ - C(O) - mit R¹ wie zuvor definiert darstellt.

Insbesondere können dabei die Gruppen R²O - in beliebigen Positionen des Rests X befindlich sein (vorzugsweise wobei mindestens eine Gruppe R²O - endständig ist).

Insbesondere kann gemäß einer besonderen Ausführungsform in der vorstehenden allgemeinen Formel (IIIa) R², unabhängig voneinander, Wasserstoff oder einen Rest CH₃ - CH(OH) - CH₂ - C(O) - darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OH) - CH₂ - C(O) - darstellt.

Weiterhin kann gemäß einer anderen besonderen Ausführungsform in der allgemeinen Formel (IIIa) R², unabhängig voneinander, Wasserstoff oder einen Rest CH₃ - CH(OR¹) - CH₂ - C(O) - mit R¹ wie zuvor definiert darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OR¹) - CH₂ - C(O) - mit R¹ wie zuvor definiert darstellt.

Des Weiteren kann gemäß einer wiederum anderen besonderen Ausführungsform in der allgemeinen Formel (IIIa) R², unabhängig voneinander, darstellen: einen Rest CH₃ - CH(OH) - CH₂ - C(O) - oder einen Rest CH₃ - CH(OR¹) - CH₂ - C(O) - mit R¹ wie zuvor definiert.

Insbesondere können bei dem erfindungsgemäßen Herstellungsverfahren als Reaktionsprodukt (III) ein oder mehrere Polyolester, insbesondere Polyglycerinester, von gegebenenfalls acylierter 3-Hydroxybuttersäure der allgemeinen Formel (IIIb)

R²O - CH₂ - CH(OR²) - CH₂ - [O - CH₂ - CH(OR²) - CH₂]ₚ - OR² (IIIb)

erhalten werden,
wobei in der allgemeinen Formel (IIIb)
- die Variable p eine ganze Zahl von 1 bis 4, insbesondere 1 oder 2, vorzugsweise 1, darstellt,
- R², unabhängig voneinander, darstellt: Wasserstoff, einen Rest CH₃ - CH(OH) - CH₂ - C(O) - oder einen Rest CH₃ - CH(OR¹) - CH₂ - C(O) - mit R¹ = Acylgruppe, ausgewählt aus - C(O) - CH₃ (Acetylgruppe) oder - C(O) - C₂H₅ (Propionylgruppe), bevorzugt - C(O) - CH₃ (Acetylgruppe), jedoch mit der Maßgabe, dass mindestens ein Rest R², insbesondere mindestens zwei Reste R², keinen Wasserstoff darstellt, und/oder mit der Maßgabe, dass mindestens ein Rest R², insbesondere mindestens zwei Reste R², einen Rest CH₃ - CH(OH) - CH₂ - C(O) - oder einen Rest CH₃ - CH(OR¹) - CH₂ - C(O) - mit R¹ wie zuvor definiert darstellt.

Insbesondere kann gemäß einer besonderen Ausführungsform in der vorstehenden allgemeinen Formel (IIIb) R², unabhängig voneinander, Wasserstoff oder einen Rest CH₃ - CH(OH) - CH₂ - C(O) - darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OH) - CH₂ - C(O) - darstellt.

Weiterhin kann gemäß einer anderen besonderen Ausführungsform in der allgemeinen Formel (IIIb) R², unabhängig voneinander, Wasserstoff oder einen Rest CH₃ - CH(OR¹) - CH₂ - C(O) - mit R¹ wie zuvor definiert darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OR¹) - CH₂ - C(O) - mit R¹ wie zuvor definiert darstellt.

Des Weiteren kann gemäß einer wiederum anderen besonderen Ausführungsform in der allgemeinen Formel (IIIb) R², unabhängig voneinander, darstellen: einen Rest CH₃ - CH(OH) - CH₂ - C(O) - oder einen Rest CH₃ - CH(OR¹) - CH₂ - C(O) - mit R¹ wie zuvor definiert.

Gemäß einer besonderen Ausführungsform des erfindungsgemäßen Verfahrens können als Reaktionsprodukt (III) insbesondere ein oder mehrere Polyolester, insbesondere Polyglycerinester, von gegebenenfalls acylierter 3-Hydroxybuttersäure der allgemeinen Formel (IIIc)

R²O - CH₂ - CH(OR²) - CH₂ - O - CH₂ - CH(OR²) - CH₂ - OR² (IIIc)

erhalten werden,
wobei in der allgemeinen Formel (IIIc) R², unabhängig voneinander, darstellt: Wasserstoff, einen Rest CH₃ - CH(OH) - CH₂ - C(O) - oder einen Rest CH₃ - CH(OR¹) - CH₂ - C(O) - mit R¹ = Acylgruppe, ausgewählt aus - C(O) - CH₃ (Acetylgruppe) oder - C(O) - C₂H₅ (Propionylgruppe), bevorzugt - C(O) - CH₃ (Acetylgruppe), jedoch mit der Maßgabe, dass mindestens ein Rest R², insbesondere mindestens zwei Reste R², keinen Wasserstoff darstellt, und/oder mit der Maßgabe, dass mindestens ein Rest R², insbesondere mindestens zwei Reste R², einen Rest CH₃ - CH(OH) - CH₂ - C(O) - oder einen Rest CH₃ - CH(OR¹) - CH₂ - C(O) - mit R¹ wie zuvor definiert darstellt.

Insbesondere kann gemäß einer besonderen Ausführungsform in der allgemeinen Formel (IIIc) R², unabhängig voneinander, Wasserstoff oder einen Rest CH₃ - CH(OH) - CH₂ - C(O) - darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OH) - CH₂ - C(O) - darstellt. Weiterhin kann gemäß einer anderen besonderen Ausführungsform in der allgemeinen Formel (IIIc) R², unabhängig voneinander, Wasserstoff oder einen Rest CH₃ - CH(OR¹) - CH₂ - C(O) - mit R¹ wie zuvor definiert darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OR¹) - CH₂ - C(O) - mit R¹ wie zuvor definiert darstellt.

Des Weiteren kann gemäß einer wiederum anderen besonderen Ausführungsform in der allgemeinen Formel (IIIc) R², unabhängig voneinander, darstellen: einen Rest CH₃ - CH(OH) - CH₂ - C(O) - oder einen Rest CH₃ - CH(OR¹) - CH₂ - C(O) - mit R¹ wie zuvor definiert.

Gemäß einer weiteren besonderen Ausführungsform des erfindungsgemäßen Verfahrens kann als Reaktionsprodukt (III) ein Gemisch von mindestens zwei voneinander verschiedenen Polyolestern, insbesondere Polyglycerinestern, von gegebenenfalls acylierter 3-Hydroxybuttersäure, insbesondere wie zuvor definiert, erhalten werden.

Gemäß einer weiteren besonderen Ausführungsform des erfindungsgemäßen Verfahrens kann als Reaktionsprodukt (III) ein Gemisch von mindestens drei voneinander verschiedenen Polyolestern, insbesondere Polyglycerinestern, von gegebenenfalls acylierter 3-Hydroxybuttersäure, insbesondere wie zuvor definiert, erhalten werden.

Wie zuvor bereits ausgeführt, wird üblicherweise das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*)*.* Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Eine erfindungsgemäß besonders bevorzugte Vorgehensweise wird durch das nachfolgende Reaktions- bzw. Syntheseschema veranschaulicht (wobei in Abhängigkeit von der Reaktionsführung entweder einzelne der Ester oder ein Gemisch von zwei oder mehreren hiervon erhalten werden):

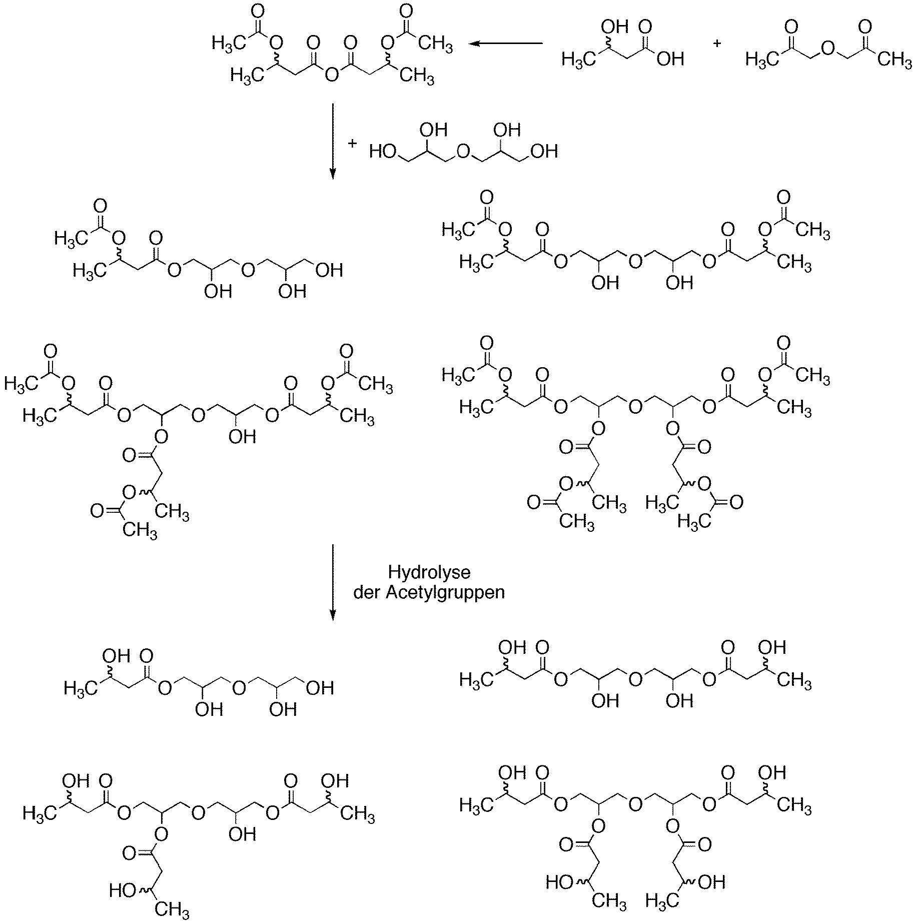

Wie zuvor ausgeführt, kann sich dem vorstehenden Reaktions- bzw. Syntheseschema optional auch noch eine wie zuvor beschriebene Funktionalisierung noch verbliebener bzw. noch freier OH-Gruppen anschließen. Weiterer Gegenstand - gemäß einem **zweiten** Aspekt der vorliegenden Erfindung - ist das nach dem erfindungsgemäßen Verfahren erhältliche bzw. erfindungsgemäße Reaktionsprodukt (d. h. ein oder mehrere Polyolester, insbesondere Polyglycerinester, von gegebenenfalls acylierter 3-Hydroxybuttersäure oder deren Gemische), nämlich ein Polyolester, insbesondere Polyglycerinester, von gegebenenfalls acylierter 3-Hydroxybuttersäure,
wobei der Polyolester der allgemeinen Formel (IIIb)

   R²O - CH₂ - CH(OR²) - CH₂ - [O - CH₂ - CH(OR²) - CH₂]ₚ - OR² (IIIb)

   entspricht,
wobei in der allgemeinen Formel (IIIb)
   - die Variable p eine ganze Zahl von 1 bis 4, insbesondere 1 oder 2, vorzugsweise 1, darstellt,
   - R², unabhängig voneinander, darstellt: Wasserstoff, einen Rest CH₃ - CH(OH) - CH₂ - C(O) - oder einen Rest CH₃ - CH(OR¹) - CH₂ - C(O) - mit R¹ = Acylgruppe, ausgewählt aus - C(O) - CH₃ (Acetylgruppe) oder - C(O) - C₂H₅ (Propionylgruppe), bevorzugt - C(O) - CH₃ (Acetylgruppe), jedoch mit der Maßgabe, dass mindestens ein Rest R², insbesondere mindestens zwei Reste R², keinen Wasserstoff darstellt, und/oder mit der Maßgabe, dass mindestens ein Rest R², insbesondere mindestens zwei Reste R², einen Rest CH₃ - CH(OH) - CH₂ - C(O) - oder einen Rest CH₃ - CH(OR¹) - CH₂ - C(O) - mit R¹ wie zuvor definiert darstellt.

Das erfindungsgemäße Reaktionsprodukt (III) umfasst also einen oder mehrere Polyolester, insbesondere Polyglycerinester, von gegebenenfalls acylierter 3-Hydroxybuttersäure der allgemeinen Formel (IIIb)

R²O - CH₂ - CH(OR²) - CH₂ - [O - CH₂ - CH(OR²) - CH₂]ₚ - OR² (IIIb)

wobei in der allgemeinen Formel (IIIb)
- die Variable p eine ganze Zahl von 1 bis 4, insbesondere 1 oder 2, vorzugsweise 1, darstellt,
- R², unabhängig voneinander, darstellt: Wasserstoff, einen Rest CH₃ - CH(OH) - CH₂ - C(O) - oder einen Rest CH₃ - CH(OR¹) - CH₂ - C(O) - mit R¹ = Acylgruppe, ausgewählt aus - C(O) - CH₃ (Acetylgruppe) oder - C(O) - C₂H₅ (Propionylgruppe), bevorzugt - C(O) - CH₃ (Acetylgruppe), jedoch mit der Maßgabe, dass mindestens ein Rest R², insbesondere mindestens zwei Reste R², keinen Wasserstoff darstellt, und/oder mit der Maßgabe, dass mindestens ein Rest R², insbesondere mindestens zwei Reste R², einen Rest CH₃ - CH(OH) - CH₂ - C(O) - oder einen Rest CH₃ - CH(OR¹) - CH₂ - C(O) - mit R¹ wie zuvor definiert darstellt.

Insbesondere kann gemäß einer besonderen Ausführungsform in diesem Zusammenhang in der allgemeinen Formel (IIIb) R², unabhängig voneinander, Wasserstoff oder einen Rest CH₃ - CH(OH) - CH₂ - C(O) - darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OH) - CH₂ - C(O) - darstellt.

Weiterhin kann gemäß einer anderen besonderen Ausführungsform in der allgemeinen Formel (IIIb) R², unabhängig voneinander, Wasserstoff oder einen Rest CH₃ - CH(OR¹) - CH₂ - C(O) - mit R¹ wie zuvor definiert darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OR¹) - CH₂ - C(O) - mit R¹ wie zuvor definiert darstellt.

Des Weiteren kann gemäß einer wiederum anderen besonderen Ausführungsform in der allgemeinen Formel (IIIb) R², unabhängig voneinander, darstellen: einen Rest CH₃ - CH(OH) - CH₂ - C(O) - oder einen Rest CH₃ - CH(OR¹) - CH₂ - C(O) - mit R¹ wie zuvor definiert.

Gemäß einer besonderen Ausführungsform der vorliegenden Erfindung kann das Reaktionsprodukt (III) insbesondere ein oder mehrere Polyolester, insbesondere Polyglycerinester, von gegebenenfalls acylierter 3-Hydroxybuttersäure der allgemeinen Formel (IIIc)

R²O - CH₂ - CH(OR²) - CH₂ - O - CH₂ - CH(OR²) - CH₂ - OR² (IIIc)

umfassen,
wobei in der allgemeinen Formel (IIIc) R², unabhängig voneinander, darstellt: Wasserstoff, einen Rest CH₃ - CH(OH) - CH₂ - C(O) - oder einen Rest CH₃ - CH(OR¹) - CH₂ - C(O) - mit R¹ = Acylgruppe, ausgewählt aus - C(O) - CH₃ (Acetylgruppe) oder - C(O) - C₂H₅ (Propionylgruppe), bevorzugt - C(O) - CH₃ (Acetylgruppe), jedoch mit der Maßgabe, dass mindestens ein Rest R², insbesondere mindestens zwei Reste R², keinen Wasserstoff darstellt, und/oder mit der Maßgabe, dass mindestens ein Rest R², insbesondere mindestens zwei Reste R², einen Rest CH₃ - CH(OH) - CH₂ - C(O) - oder einen Rest CH₃ - CH(OR¹) - CH₂ - C(O) - mit R¹ wie zuvor definiert darstellt.

Insbesondere kann gemäß einer besonderen Ausführungsform in diesem Zusammenhang in der allgemeinen Formel (IIIc) R², unabhängig voneinander, Wasserstoff oder einen Rest CH₃ - CH(OH) - CH₂ - C(O) - darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OH) - CH₂ - C(O) - darstellt.

Weiterhin kann gemäß einer anderen besonderen Ausführungsform in der allgemeinen Formel (IIIc) R², unabhängig voneinander, Wasserstoff oder einen Rest CH₃ - CH(OR¹) - CH₂ - C(O) - mit R¹ wie zuvor definiert darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OR¹) - CH₂ - C(O) - mit R¹ wie zuvor definiert darstellt.

Des Weiteren kann gemäß einer wiederum anderen besonderen Ausführungsform in der allgemeinen Formel (IIIc) R², unabhängig voneinander, darstellen: einen Rest CH₃ - CH(OH) - CH₂ - C(O) - oder einen Rest CH₃ - CH(OR¹) - CH₂ - C(O) - mit R¹ wie zuvor definiert.

Gemäß einer weiteren besonderen Ausführungsform kann das Reaktionsprodukt (III) insbesondere ein Gemisch von mindestens zwei voneinander verschiedenen Polyolestern, insbesondere Polyglycerinestern, von gegebenenfalls acylierter 3-Hydroxybuttersäure, insbesondere wie zuvor definiert, umfassen (d. h. als Reaktionsprodukt (III) wird gemäß dieser Ausführungsform ein Gemisch von mindestens zwei voneinander verschiedenen Polyolestern, insbesondere Polyglycerinestern, von gegebenenfalls acylierter 3-Hydroxybuttersäure, insbesondere wie zuvor definiert, erhalten).

Gemäß einer weiteren besonderen Ausführungsform kann das Reaktionsprodukt (III) insbesondere ein Gemisch von mindestens drei voneinander verschiedenen Polyolestern, insbesondere Polyglycerinestern, von gegebenenfalls acylierter 3-Hydroxybuttersäure, insbesondere wie zuvor definiert, umfassen (d. h. als Reaktionsprodukt (III) wird gemäß dieser Ausführungsform ein Gemisch von mindestens drei voneinander verschiedenen Polyolestern, insbesondere Polyglycerinestern, von gegebenenfalls acylierter 3-Hydroxybuttersäure, insbesondere wie zuvor definiert, erhalten).

Gegenstand der vorliegenden Erfindung ist somit ebenfalls ein Polyolester, insbesondere Polyglycerinester, von gegebenenfalls acylierter 3-Hydroxybuttersäure, insbesondere ein wie zuvor beschriebener bzw. definierter Polyolester, wobei der Polyolester der allgemeinen Formel (IIIb)

R²O - CH₂ - CH(OR²) - CH₂ - [O - CH₂ - CH(OR²) - CH₂]ₚ - OR² (IIIb)

entspricht,
wobei in der allgemeinen Formel (IIIb)
- die Variable p eine ganze Zahl von 1 bis 4, insbesondere 1 oder 2, vorzugsweise 1, darstellt,
- R², unabhängig voneinander, darstellt: Wasserstoff, einen Rest CH₃ - CH(OH) - CH₂ - C(O) - oder einen Rest CH₃ - CH(OR¹) - CH₂ - C(O) - mit R¹ = Acylgruppe, ausgewählt aus - C(O) - CH₃ (Acetylgruppe) oder - C(O) - C₂H₅ (Propionylgruppe), bevorzugt - C(O) - CH₃ (Acetylgruppe), jedoch mit der Maßgabe, dass mindestens ein Rest R², insbesondere mindestens zwei Reste R², keinen Wasserstoff darstellt, und/oder mit der Maßgabe, dass mindestens ein Rest R², insbesondere mindestens zwei Reste R², einen Rest CH₃ - CH(OH) - CH₂ - C(O) - oder einen Rest CH₃ - CH(OR¹) - CH₂ - C(O) - mit R¹ wie zuvor definiert darstellt.

Insbesondere kann gemäß einer besonderen Ausführungsform in der vorstehenden allgemeinen Formel (IIIb) R², unabhängig voneinander, Wasserstoff oder einen Rest CH₃ - CH(OH) - CH₂ - C(O) - darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OH) - CH₂ - C(O) - darstellt.

Weiterhin kann gemäß einer anderen besonderen Ausführungsform in der allgemeinen Formel (IIIb) R², unabhängig voneinander, Wasserstoff oder einen Rest CH₃ - CH(OR¹) - CH₂ - C(O) - mit R¹ wie zuvor definiert darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OR¹) - CH₂ - C(O) - mit R¹ wie zuvor definiert darstellt.

Des Weiteren kann gemäß einer wiederum anderen besonderen Ausführungsform in der allgemeinen Formel (IIIb) R², unabhängig voneinander, darstellen: einen Rest CH₃ - CH(OH) - CH₂ - C(O) - oder einen Rest CH₃ - CH(OR¹) - CH₂ - C(O) - mit R¹ wie zuvor definiert.

Wiederum weiterer Gegenstand der vorliegenden Erfindung ist ebenfalls ein Polyolester, insbesondere Polyglycerinester, von gegebenenfalls acylierter 3-Hydroxybuttersäure, insbesondere ein wie zuvor beschriebener bzw. definierter Polyolester,
wobei der Polyolester der allgemeinen Formel (IIIc),

   R²O - CH₂ - CH(OR²) - CH₂ - O - CH₂ - CH(OR²) - CH₂ - OR² (IIIc)
entspricht, wobei in der allgemeinen Formel (IIIc) R², unabhängig voneinander, darstellt: Wasserstoff, einen Rest CH₃- CH(OH) - CH₂ - C(O) - oder einen Rest CH₃ - CH(OR¹) - CH₂ - C(O) - mit R¹ = Acylgruppe, ausgewählt aus - C(O) - CH₃ (Acetylgruppe) oder - C(O) - C₂H₅ (Propionylgruppe), bevorzugt - C(O) - CH₃ (Acetylgruppe), jedoch mit der Maßgabe, dass mindestens ein Rest R², insbesondere mindestens zwei Reste R², keinen Wasserstoff darstellt, und/oder mit der Maßgabe, dass mindestens ein Rest R², insbesondere mindestens zwei Reste R², einen Rest CH₃ - CH(OH) - CH₂ - C(O) - oder einen Rest CH₃ - CH(OR¹) - CH₂ - C(O) - mit R¹ wie zuvor definiert darstellt.

Insbesondere kann gemäß einer besonderen Ausführungsform in der allgemeinen Formel (IIIc) R², unabhängig voneinander, Wasserstoff oder einen Rest CH₃ -CH(OH) - CH₂ - C(O) - darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OH) - CH₂ - C(O) - darstellt.

Weiterhin kann gemäß einer anderen besonderen Ausführungsform in der allgemeinen Formel (IIIc) R², unabhängig voneinander, Wasserstoff oder einen Rest CH₃ - CH(OR¹) - CH₂ - C(O) - mit R¹ wie zuvor definiert darstellen, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OR¹) - CH₂ - C(O) - mit R¹ wie zuvor definiert darstellt.

Des Weiteren kann gemäß einer wiederum anderen besonderen Ausführungsform in der allgemeinen Formel (IIIc) R², unabhängig voneinander, darstellen: einen Rest CH₃ - CH(OH) - CH₂ - C(O) - oder einen Rest CH₃ - CH(OR¹) - CH₂ - C(O) - mit R¹ wie zuvor definiert.

Weiterer Gegenstand der vorliegenden Erfindung gemäß diesem Erfindungsaspekt ist entsprechend einer besonderen Ausführungsform ein Gemisch, welches mindestens zwei voneinander verschiedene Polyolester, insbesondere Polyglycerinester, von gegebenenfalls acylierter 3-Hydroxybuttersäure, wie zuvor definiert, umfasst.

Wiederum weiterer Gegenstand der vorliegenden Erfindung gemäß diesem Erfindungsaspekt ist entsprechend einer weiteren besonderen Ausführungsform ein Gemisch, welches mindestens drei voneinander verschiedene Polyolester, insbesondere Polyglycerinester, von gegebenenfalls acylierter 3-Hydroxybuttersäure, wie zuvor definiert, umfasst.

Das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Polyolester, insbesondere Polyglycerinester, von gegebenenfalls acylierter 3-Hydroxybuttersäure, wie zuvor definiert, und/oder das nach dem erfindungsgemäßen Herstellungsverfahren erhältliche bzw. erfindungsgemäße Gemisch, wie zuvor definiert, weist gegenüber dem Stand der Technik eine Vielzahl von Vorteilen und Besonderheiten auf:
Wie die Anmelderin überraschend herausgefunden hat, eignet sich das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Polyolester, insbesondere Polyglycerinester, von gegebenenfalls acylierter 3-Hydroxybuttersäure, wie zuvor definiert, und/oder das nach dem erfindungsgemäßen Herstellungsverfahren erhältliche bzw. erfindungsgemäße Gemisch, wie zuvor definiert, insbesondere als Präkursor bzw. Metabolit von 3-Hydroxybuttersäure bzw. deren Salzen, da dieser bzw. dieses einerseits physiologisch, insbesondere im Magen/Darm-Trakt, zu 3-Hydroxybuttersäure bzw. deren Salzen umgesetzt wird und andererseits gleichzeitig eine gute physiologische Kompatibilität bzw. Verträglichkeit aufweist, insbesondere im Hinblick auf Nichttoxizität und akzeptable organoleptische Eigenschaften.

Darüber hinaus ist das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Polyolester, insbesondere Polyglycerinester, von gegebenenfalls acylierter 3-Hydroxybuttersäure, wie zuvor definiert, und/oder das nach dem erfindungsgemäßen Herstellungsverfahren erhältliche bzw. erfindungsgemäße Gemisch, wie zuvor definiert, ohne Weiteres auf synthetischem Wege auch in großtechnischem Maßstab zugänglich bzw. verfügbar, und zwar auch mit der erforderlichen pharmazeutischen bzw. pharmakologischen Qualität.

Zudem kann das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Polyolester, insbesondere Polyglycerinester, von gegebenenfalls acylierter 3-Hydroxybuttersäure, wie zuvor definiert, und/oder das nach dem erfindungsgemäßen Herstellungsverfahren erhältliche bzw. erfindungsgemäße Gemisch, wie zuvor definiert, erforderlichenfalls in enantiomerenreiner bzw. enantiomerenangereicherter Form bereitgestellt werden.

Das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Polyolester, insbesondere Polyglycerinester, von gegebenenfalls acylierter 3-Hydroxybuttersäure, wie zuvor definiert, und/oder das nach dem erfindungsgemäßen Herstellungsverfahren erhältliche bzw. erfindungsgemäße Gemisch, wie zuvor definiert, stellt somit ein effizientes pharmakologisches Wirkstoff-Target im Rahmen einer Ketokörper-Therapie des menschlichen oder tierischen Körpers dar.

Nachfolgend werden noch die übrigen Erfindungsaspekte weiterführende erläutert und im Detail beschrieben.

Weiterer Gegenstand der vorliegenden Erfindung - gemäß einem **dritten** Aspekt der vorliegenden Erfindung - ist eine pharmazeutische Zusammensetzung, insbesondere ein Arzneimittel oder Medikament, welche(s) einen nach dem erfindungsgemäßen Herstellungsverfahren erhältlichen bzw. erfindungsgemäßen Polyolester, insbesondere Polyglycerinester, von gegebenenfalls acylierter 3-Hydroxybuttersäure, wie zuvor definiert, und/oder ein nach dem erfindungsgemäßen Herstellungsverfahren erhältliches bzw. erfindungsgemäßes Gemisch, wie zuvor definiert, umfasst.

Insbesondere betrifft die vorliegende Erfindung gemäß diesem Erfindungsaspekt eine pharmazeutische Zusammensetzung zur prophylaktischen und/oder therapeutischen Behandlung bzw. zur Verwendung bei der prophylaktischen und/oder therapeutischen Behandlung von Erkrankungen des menschlichen oder tierischen Körpers. Hierbei kann es sich insbesondere um Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Wiederum weiterer Gegenstand der vorliegenden Erfindung - gemäß einem **vierten** Aspekt der vorliegenden Erfindung - ist ein nach dem erfindungsgemäßen Herstellungsverfahren erhältlicher bzw. erfindungsgemäßer Polyolester, insbesondere Polyglycerinester, von gegebenenfalls acylierter 3-Hydroxybuttersäure, wie zuvor definiert, und/oder ein nach dem erfindungsgemäßen Herstellungsverfahren erhältliches bzw. erfindungsgemäßes Gemisch, wie zuvor definiert, zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen weiterer Gegenstand der vorliegenden Erfindung - gemäß einem **fünften** Aspekt der vorliegenden Erfindung - ist die Verwendung eines nach dem erfindungsgemäßen Herstellungsverfahren erhältlichen bzw. erfindungsgemäßen Polyolesters, insbesondere Polyglycerinesters, von gegebenenfalls acylierter 3-Hydroxybuttersäure, wie zuvor definiert, und/oder eines nach dem erfindungsgemäßen Herstellungsverfahren erhältlichen bzw. erfindungsgemäßen Gemischs, wie zuvor definiert, zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen weiterer Gegenstand der vorliegenden Erfindung - gemäß einem **sechsten** Aspekt der vorliegenden Erfindung - ist die Verwendung eines nach dem erfindungsgemäßen Herstellungsverfahren erhältlichen bzw. erfindungsgemäßen Polyolesters, insbesondere Polyglycerinesters, von gegebenenfalls acylierter 3-Hydroxybuttersäure, wie zuvor definiert, und/oder eines nach dem erfindungsgemäßen Herstellungsverfahren erhältlichen bzw. erfindungsgemäßen Gemischs, wie zuvor definiert, zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung.

Gleichermaßen weiterer Gegenstand der vorliegenden Erfindung - gemäß einem **siebten** Aspekt der vorliegenden Erfindung - ist ein Nahrungsmittel- und/oder Lebensmittelerzeugnis, welches einen nach dem erfindungsgemäßen Herstellungsverfahren erhältlichen bzw. erfindungsgemäßen Polyolester, insbesondere Polyglycerinester, von gegebenenfalls acylierter 3-Hydroxybuttersäure, wie zuvor definiert, und/oder ein nach dem erfindungsgemäßen Herstellungsverfahren erhältliches bzw. erfindungsgemäßes Gemisch, wie zuvor definiert, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel (*Functional Food*)*,* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Schließlich ist wiederum weiterer Gegenstand der vorliegenden Erfindung - gemäß einem **achten** Aspekt der vorliegenden Erfindung - die Verwendung eines nach dem erfindungsgemäßen Herstellungsverfahren erhältlichen bzw. erfindungsgemäßen Polyolesters insbesondere Polyglycerinesters, von gegebenenfalls acylierter 3-Hydroxybuttersäure, wie zuvor definiert, und/oder eines nach dem erfindungsgemäßen Herstellungsverfahren erhältlichen Gemischs, wie zuvor definiert, in einem Nahrungsmittel- und/oder Lebensmittelerzeugnis.

Gemäß diesem Erfindungsaspekt kann das Nahrungsmittel- und/oder Lebensmittelerzeugnis insbesondere ein Nahrungsergänzungsmittel, ein funktionelles Lebensmittel (*Functional Food*)*,* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Weitere Ausgestaltungen, Abwandlungen und Variationen der vorliegenden Erfindung sind für den Fachmann beim Lesen der Beschreibung ohne Weiteres erkennbar oder realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen, sondern lediglich die beispielhafte und nichtlimitierende Durchführungsweise und Ausgestaltung der vorliegenden Erfindung erläutern sollen.

### AUSFÜHRUNGSBEISPIELE:

### Verwendete Abkürzungen

BHB = 3-BHB = 3-Hydroxybuttersäure
PG(2) = Diglycerin: HO - CH₂ - CH(OH) - CH₂ - O - CH₂ - CH(OH) - CH₂ - OH
PG(3) = Polyglycerin: HO - CH₂ - CH(OH) - CH₂ - [O - CH₂ - CH(OH) - CH₂]₂ - OH

### Herstellungsbeispiele

Das erfindungsgemäße Herstellungsverfahren wird anhand der nachfolgenden Ausführungsbeispiele veranschaulicht. Das diesbezügliche allgemeine Reaktionsschema hierzu ist im allgemeinen Beschreibungsteil dargestellt und erläutert.

### Beispiel 1

### Herstellung von 3-Acetoxy-BHB-Diglycerin-Gemischen aus Diglycerin und acetyliertem 3-Hydroxybuttersäureanhydrid (= 3-Acetoxybuttersäureanhydrid) mit nachfolgender Hydrolyse der Acetylgruppen

In einem 1.000-ml-Mehrhalskolben mit Dephlegmator (Partialkondensator) und Destillationsbrücke werden 25 g einer (R)/(S)-3-Hydroxybuttersäure in 95 g Essigsäure vorgelegt. Zu der Reaktionsmischung werden bei 80 °C unter N₂-Atmosphäre 90 g Essigsäureanhydrid innerhalb von einer Stunde zu getropft. Das Reaktionsgemisch wird bei 80 °C für weitere 4 bis 5 Stunden gerührt. Es wird 3-Acetoxybuttersäureanhydrid (= acetyliertes 3-Hydroxybuttersäureanhydrid) gebildet.

Zu der Reaktionsmischung werden dann bei 80 °C 3,5 g Diglycerin gegeben und für 8 bis 10 Stunden gerührt. Das Reaktionsprodukt ist ein Gemisch von Diglycerinestern der 3-Acetoxybuttersäure (d. h. also mit anderen Worten ein Gemisch von Diglycerinestern der acetylierten 3-Hydroxybuttersäure).

Die gebildeten Nebenprodukte (Essigsäure aus der ersten Stufe und 3-Acetoxybuttersäure aus der zweiten Stufe) werden im Vakuum (< 50 mbar) bei 100 bis 120 °C abdestilliert. Die Charakterisierung erfolgt mittels GC, GPC und GC-MS.

Ein Teil des Reaktionsprodukts (d. h. Gemisch von Diglycerinestern der 3-Acetoxybuttersäure) wird anschließend einer Hydrolyse der Acetylgruppen unterzogen (partielle bzw. selektive Hydrolyse in Gegenwart von Enzym). Hierzu wird das Reaktionsprodukt in wässrigem Milieu in Gegenwart von immobilisiertem Enzym (CALB-Lipase auf Polymerträger, abgeleitet von *Candida antarctica,* erster Ansatz: Novozym^{®} 435 von der Fa. Sigma-Aldrich bzw. Merck und zweiter Ansatz: Lipozym^{®} 435 von der Fa. Strem Chemicals, Inc.) für 8 Stunden bei 50 °C umgesetzt. Nach Abtrennung des Enzyms und anschließender destillativer Aufreinigung wird als Hydrolyseprodukt ein betreffendes Gemisch von Diglycerinestern der 3-Hydroxybuttersäure erhalten. Die Charakterisierung erfolgt mittels GC, GPC und GC-MS.

Ein Teil des erhaltenen Gemischs der Diglycerinester der 3-Hydroxybuttersäure wird chromatographisch in die einzelnen Diglycerinester (d. h. Mono-Diglycerinester, Di-Diglycerinester, Tri-Diglycerinester etc.) aufgetrennt und die einzelnen Diglycerinester werden jeweils als Reinsubstanzen erhalten.

### Beispiel 2

### Weitere Herstellung von 3-Acetyl-BHB-Diglycerin-Gemischen aus Diglycerin und acetyliertem 3-Hydroxybuttersäureanhydrid (= 3-Acetoxybuttersäureanhydrid) mit nachfolgender Hydrolyse der Acetylgruppen

Beispiel 1 wird wiederholt, jedoch wird nach der Umsetzung von (R)/(S)-3-Hydroxybuttersäureanhydrid das gebildete Nebenprodukt (Essigsäure) im Vakuum (< 50 mbar) bei 100 bis 120 °C destillativ entfernt und es wird reines 3-Acetoxybuttersäureanhydrid erhalten. Die Charakterisierung erfolgt mittels GC, GPC und GC-MS.

Anschließend wird das reine 3-Acetoxybuttersäureanhydrid mit Diglycerin umgesetzt, aufgereinigt und analysiert (wie in Beispiel 1 beschrieben), sodass ein reines Gemisch von Diglycerinestern der 3-Acetoxybuttersäure erhalten wird.

Ein Teil des Reaktionsprodukts (d. h. Gemisch von Diglycerinestern der 3-Acetoxybuttersäure) wird anschließend, wie in Beispiel 1 beschrieben, hydrolysiert, sodass ein Gemisch von Diglycerinestern der 3-Hydroxybuttersäure erhalten wird. Die Charakterisierung erfolgt mittels GC, GPC und GC-MS.

Ein Teil des erhaltenen Gemischs der Diglycerinester der 3-Hydroxybuttersäure wird chromatographisch in die einzelnen Diglycerinester (d. h. Mono-Diglycerinester, Di-Diglycerinester, Tri-Diglycerinester etc.) aufgetrennt und die einzelnen Diglycerinester werden jeweils als Reinsubstanzen erhalten.

### Beispiel 3

### Weitere Herstellung von 3-Acetyl-BHB-Diglycerin-Gemischen aus Diglycerin und acetyliertem 3-Hydroxybuttersäureanhydrid (= 3-Acetoxybuttersäureanhydrid) mit nachfolgender Hydrolyse der Acetylgruppen

In einem 5.000-ml-Mehrhalskolben mit Dephlegmator (Partialkondensator) und Destillationsbrücke werden 250 g einer (R)/(S)-3-Hydroxybuttersäure in 950 g Essigsäure vorgelegt. Zu der Reaktionsmischung werden bei 80 °C unter N₂-Atmosphäre 900 g Essigsäureanhydrid innerhalb von einer Stunde zu getropft. Das Reaktionsgemisch wird bei 80 °C für weitere 4 bis 5 Stunden gerührt. Es wird 3-Acetoxybuttersäureanhydrid (= acetyliertes 3-Hydroxybuttersäureanhydrid) gebildet. Das gebildete Nebenprodukt (Essigsäure) wird im Vakuum (< 50 mbar) bei 100 bis 120 °C destillativ entfernt und es wird reines 3-Acetoxybuttersäureanhydrid erhalten. Die Charakterisierung erfolgt mittels GC, GPC und GC-MS.

Zu dem aufgereinigten 3-Acetoxybuttersäureanhydrid werden dann bei 80 °C 35 g Diglycerin gegeben und für 8 bis 10 Stunden gerührt. Das Reaktionsprodukt ist ein Gemisch von Diglycerinestern der 3-Acetoxybuttersäure (d. h. also mit anderen Worten ein Gemisch von Diglycerinestern der acetylierten 3-Hydroxybuttersäure).

Das gebildete Nebenprodukt (3-Acetoxybuttersäure) wird im Vakuum (< 50 mbar) bei 100 bis 120 °C abdestilliert. Die Charakterisierung erfolgt mittels GC, GPC und GC-MS.

Ein Teil des Reaktionsprodukts (d. h. Gemisch von Diglycerinestern der 3-Acetoxybuttersäure) wird anschließend einer Hydrolyse der Acetylgruppen unterzogen (partielle bzw. selektive Hydrolyse in Gegenwart von Enzym). Hierzu wird das Reaktionsprodukt in wässrigem Milieu in Gegenwart von immobilisiertem Enzym (CALB-Lipase auf Polymerträger, abgeleitet von *Candida antarctica,* erster Ansatz: Novozym^{®} 435 von der Fa. Sigma-Aldrich bzw. Merck und zweiter Ansatz: Lipozym^{®} 435 von der Fa. Strem Chemicals, Inc.) für 8 Stunden bei 50 °C umgesetzt. Nach Abtrennung des Enzyms und anschließender destillativer Aufreinigung wird als Hydrolyseprodukt ein betreffendes Gemisch von Diglycerinestern der 3-Hydroxybuttersäure erhalten. Die Charakterisierung erfolgt mittels GC, GPC und GC-MS.

Ein Teil des erhaltenen Gemischs der Diglycerinester der 3-Hydroxybuttersäure wird chromatographisch in die einzelnen Diglycerinester (d. h. Mono-Diglycerinester, Di-Diglycerinester, Tri-Diglycerinester etc.) aufgetrennt und die einzelnen Diglycerinester werden jeweils als Reinsubstanzen erhalten.

### Weitere Herstellung von 3-BHB-Diglycerinester-Gemischen

Die drei vorangehenden Versuche werden jeweils wiederholt, jedoch mit anderen Polyolen (nämlich jeweils mit Polyglycerin PG(3) und mit 1,2-Pentandiol).

Die vorgenannten Polyalkohole 1,2-Pentandiol sowie Polyglycerin PG(3) werden autokatalytisch effizient zu den gewünschten Produkten umgesetzt. Es werden vergleichbare Ergebnisse zu den vorangehenden Versuchen erhalten. Aufreinigung, Trennung bzw. Fraktionierung und Hydrolyse erfolgen in gleicher Weise.

Die Versuche mit 1,2-Pentandiol, Diglycerin und Polyglycerin PG(3) werden mit Schwefelsäure (H₂SO₄) als Katalysator und bei Temperaturen zwischen 75 und 110 °C wiederholt. Es werden vergleichbare Ergebnisse erhalten. Aufreinigung, Trennung bzw. Fraktionierung und Hydrolyse erfolgen in gleicher Weise.

Die Versuche mit 1,2-Pentandiol, Diglycerin und Polyglycerin PG(3) werden mit Salzsäure (HCl) als Katalysator und bei Temperaturen zwischen 75 und 110 °C wiederholt. Es werden vergleichbare Ergebnisse erhalten. Aufreinigung, Trennung bzw. Fraktionierung und Hydrolyse erfolgen in gleicher Weise.

Die Versuche mit 1,2-Pentandiol, Diglycerin und Polyglycerin PG(3) werden mit Phosphorsäure (H₃PO₄) als Katalysator und bei Temperaturen zwischen 75 und 110 °C wiederholt. Es werden vergleichbare Ergebnisse erhalten. Aufreinigung, Trennung bzw. Fraktionierung und Hydrolyse erfolgen in gleicher Weise.

Da insbesondere die 3-BHB-Diglycerinester ein nur wenig bitteren Geschmack aufweisen, sind vor allem diese Ester eine effiziente Produktgruppe für eine therapeutische Anwendung. Daher wird der vorangehende Versuch autokatalytisch und mit Diglycerin als Polyalkohol in einem größeren Maßstab (2 bis 4 kg) durchgeführt.

Zunächst werden in einem Maßstab von 2 kg die stöchiometrischen Reaktionsbedingungen der vorangehenden Versuche angewendet (40 Mol-% Überschuss 3-Acetoxybuttersäureanhydrid). Nach 15 h wird ein Teil der Reaktionsmischung (ca. 200 g) für eine weitere Untersuchung entnommen. Hierbei handelt es sich um ein Mono/Di-Diglycerinester-Gemisch. Danach werden weitere ca. 2 kg 3-Acetoxybuttersäureanhydrid hinzugegeben. Die Menge entspricht einem Überschuss von 100 Mol-%, bereits berechnet auf das (R)-Enantiomer. Das Ziel ist die Herstellung eines Voll-Esters. Es ist zu erkennen, dass sich nach etwa 20 bis 30 h ein konstanter Gehalt an Di-PG(2)-Ester einstellt; der Mono-Diglycerinester Anteil fällt ab und der Tri-Diglycerinester-Anteil steigt. Weitere Analysen (GPC) zeigen, dass sich auch ein Tetra-Diglycerinester gebildet hat.

Nach Abdestillieren von überschüssiger 3-Acetoxybuttersäure wird eine Hydrolyse der Acetylgruppen (partielle bzw. selektive Hydrolyse in Gegenwart von Enzym) in wässrigem Milieu in Gegenwart von immobilisiertem Enzym (CALB-Lipase auf Polymerträger, abgeleitet von *Candida antarctica,* erster Ansatz: Novozym^{®} 435 von der Fa. Sigma-Aldrich bzw. Merck und zweiter Ansatz: Lipozym^{®} 435 von der Fa. Strem Chemicals, Inc.) für 8 Stunden bei 50 °C durchgeführt. Nach Abtrennung des Enzyms und anschließender destillativer Aufreinigung wird als Hydrolyseprodukt ein betreffendes Gemisch von Diglycerinestern der 3-Hydroxybuttersäure erhalten. Das Gemisch von Diglycerinestern der 3-Hydroxybuttersäure weist nur einen leicht bitteren Geschmack auf und ist organoleptisch akzeptabel und kompatibel.

### Funktionalisierungsversuche

Die in den vorangehenden Versuchen erhaltenen Mono-/Di-/Tri-/Tetra-Diglycerinester-Gemische bzw. deren acylierte Derivate sowie die chromatographisch hieraus abgetrennten einzelnen Diglycerinester in Reinform bzw. deren acylierte Analoge werden jeweils nachfolgend durch Umsetzung mit C₇-Anhydrid funktionalisiert, um vollständig an allen noch verbliebenen, d. h. noch freien OH-Gruppen veresterte Produkte zu erhalten. Das diesbezügliche allgemeine Reaktionsschema hierzu ist im allgemeinen Beschreibungsteil dargestellt und erläutert.

Die Versuche zeigen, dass die beabsichtigte Funktionalisierung durch Umsetzung mit C₇-Anhydrid zu den gewünschten Produkten führt (d. h. Veresterung der freien OH-Gruppen), wie durch entsprechende Analytik bestätigt.

Vergleichbare Funktionalisierungsversuche werden auch mit höheren Carbonsäureanhydriden (jeweils mit C₁₀-, C₂₀- und C₂₈-Carbonsäureanhydriden) durchgeführt und führen zu analogen Ergebnissen (d. h. Veresterung der freien OH-Gruppen), wie durch entsprechende Analytik bestätigt. Die Versuche zeigen, dass die beabsichtigte Funktionalisierung auch durch Umsetzung mit höheren Carbonsäureanhydriden (jeweils mit C₁₀-, C₂₀- und C₂₈-Carbonsäureanhydriden) zu den gewünschten Produkten führt (d. h. Veresterung der freien OH-Gruppen), wie durch entsprechende Analytik bestätigt.

### Weitere Funktionalisierungsversuche

Die in den vorangehenden Versuchen erhaltenen Mono-/Di-/Tri-/Tetra-Diglycerinester-Gemische bzw. deren acylierte Derivate sowie die chromatographisch hieraus abgetrennten einzelnen Diglycerinester in Reinform bzw. deren acylierte Analoge werden jeweils nachfolgend durch Umsetzung mit 3-Acetoxybuttersäureanhydrid funktionalisiert, um vollständig an allen noch verbliebenen, d. h. noch freien OH-Gruppen veresterte Produkte zu erhalten.

Die Versuche zeigen, dass die beabsichtigte Funktionalisierung durch Umsetzung mit 3-Acetoxybuttersäureanhydrid zu den gewünschten Produkten führt (d. h. Veresterung der freien OH-Gruppen), wie durch entsprechende Analytik bestätigt.

### Physiologische Anwendungsversuchen: in-vitro-Verdauversuche

### Verdauversuche (Spalt- bzw. Spaltungsversuche) von erfinduncscemäßen 3-BHB-DIglycerinester-Gemischen

Mittels Spaltungsversuchen wird gezeigt, dass erfindungsgemäß hergestellte 3-BHB-Diglycerinester bzw. deren Gemische sowie deren acylierte Derivate/Analog im menschlichen gastrointestinalen Trakt gespalten werden können.

Als Ausgangsgemisch werden jeweils eingesetzt:
- ein aufgereinigtes, nach dem erfindungsgemäßen Verfahren erhaltenes Gemisch aus 3-BHB-Monodiglycerinester, 3-BHB-Di-Diglycerinester, 3-BHB-Tri-Diglycerinester und 3-BHB-Tetra-Diglycerinester (Probe 1)
- ein aufgereinigtes, nach dem erfindungsgemäßen Verfahren erhaltenes Gemisch aus Monodiglycerinester, Di-Diglycerinester, Tri-Diglycerinester und Tetra-Diglycerinester von acylierter 3-Hydroxybuttersäure (= 3-Acetoxybuttersäure) (Probe 2)
- 3-BHB-Mono-Diglycerinester (Probe 3)
- 3-BHB-Di-Diglycerinester (Probe 4)
- 3-BHB-Tri-Diglycerinester (Probe 5)
- Mono-Diglycerinester von acylierter 3-Hydroxybuttersäure (= 3-Acetoxybuttersäure) (Probe 6)
- Di-Diglycerinester von acylierter 3-Hydroxybuttersäure (= 3-Acetoxybuttersäure) (Probe 7)
- Tri-Diglycerinester von acylierter 3-Hydroxybuttersäure (= 3-Acetoxybuttersäure) (Probe 8)
- C₇-Anhydrid-funktionalisiertes Gemisch aus 3-BHB-Monodiglycerinester, 3-BHB-Di-Diglycerinester, 3-BHB-Tri-Diglycerinester und 3-BHB-Tetra-Diglycerinester (Probe 9)
- 3-Acetoxybuttersäureanhydrid-funktionalisiertes Gemisch aus 3-BHB-Mono-diglycerinester, 3-BHB-Di-Diglycerinester, 3-BHB-Tri-Diglycerinester und 3-BHB-Tetra-Diglycerinester (Probe 10)

Für die Spaltungsversuche unter köpernahen Bedingungen werden jeweils zwei Medien für alle vorgenannten Proben 1 bis 10 untersucht:
- FaSSGF, welches den Magen simuliert
- FaSSIF, welches den Darmtrakt simuliert

Beide Medien stammen von der Firma Biorelevant^{®}, Ltd., Großbritannien. Zusätzlich wird in einigen Experimenten beiden Medien jeweils Schweine-Pankrease zugesetzt (Panzytrat^{®} 40.000, Fa. Allergan).

Die Ergebnisse der Spaltungsversuche in einem FaSSGF- bzw. FaSSIF-Medium mit Panzytrat^{®} und ohne Panzytrat^{®} (jeweils 35 °C, 24 h) zeigen, dass alle Proben unter FaSSGF-Bedingungen mit Panzytrat^{®} und ohne Panzytrat^{®} hydrolysieren; dies liegt hauptsächlich am niedrigen pH-Wert (pH = 1,6) des Mediums. Bei FaSSIF-Bedingungen findet eine geringere Umsetzung unter Verwendung von Panzytrat^{®} statt.

Bei allen Experimenten ist zu erkennen, dass sich die Kaskade (Tetraester wird zu Triester, Triester wird zu Diester etc.) bis zu der gewünschten freien Säure 3-BHB fortsetzt.

### Weitere Verdauversuche (Spaltungsversuche) von erfindunasaemäßen 3-BHB-PG(2)-Ester-Gemischen

### Spaltungsversuche mit Pankreatin

Jeweils 2 g der zuvor beschriebenen Proben 1 bis 10 werden in 50 g Wasser gelöst und mit 0,5 g (1 Gew.-%) Pankreatin versetzt. Das Pankreatin wird in Form des kommerziell verfügbaren Produkts Panzytrat^{®} 40.000 von der Fa. Allergan eingesetzt. Das Ganze wird auf einer Heizplatte bei 50 °C gerührt; der Reaktionsverlauf wird mittels kontinuierlicher Erfassung der Säurezahl über die Zeit ermittelt und verfolgt. Die Säurezahl steigt über den Beobachtungszeitraum an (Spaltung der Ester bzw. Ester-Gemische zu der freien Säure). Der Umsatz/ZeitVerlauf der wässrigen Spaltung mittels Pankreatin, einschließlich Zunahme der Säurezahl über die Zeit, belegt die gewünschte Zersetzung des Eduktgemischs zu der freien Säure. Dies wird durch entsprechende Analytik bestätigt. Die Versuche belegen, dass die erfindungsgemäßen Ausgangsproben jeweils geeignete physiologische Präkursoren von 3-Hydroxybuttersäure für die entsprechenden Ketokörpertherapien darstellt.

Die zuvor geschilderten Spaltungsversuche belegen, dass die Polyolester, insbesondere Polyglycerinester, der 3-Hydroxybuttersäure effiziente Präkursoren bzw. Metabolite der freien Hydroxybuttersäure bzw. deren Salzen darstellen, insbesondere im Hinblick auf ihre beabsichtigte Wirkung, welche in physiologisch verträglicher bzw. physiologisch kompatibler Form vorliegen.

## Patentansprüche

1. Verfahren zur Herstellung von Polyolestern, insbesondere Polyglycerinestern, von gegebenenfalls acylierter 3-Hydroxybuttersäure (beta-Hydroxybuttersäure, BHB bzw. 3-BHB),
wobei mindestens eine Verbindung der allgemeinen Formel (I)
CH₃ - CH(OR¹) - CH₂ - C(O) - O - C(O) - CH₂ - CH(OR¹)- CH₃ (I)
wobei in der allgemeinen Formel (I) der Rest R¹ eine Acylgruppe, ausgewählt aus - C(O) - CH₃ (Acetylgruppe) oder - C(O) - C₂H₅ (Propionylgruppe), bevorzugt - C(O) - CH₃ (Acetylgruppe), darstellt,
mit mindestens einem mindestens zwei Hydroxylgruppen (OH-Gruppen) enthaltenden Polyol (II), insbesondere Polyglycerin, umgesetzt wird, gegebenenfalls gefolgt von einer Hydrolyse der Acylgruppen,
so dass als Reaktionsprodukt (III) ein oder mehrere Polyolester, insbesondere Polyglycerinester, von gegebenenfalls acylierter 3-Hydroxybuttersäure erhalten werden.

2. Verfahren nach Anspruch 1,
wobei die Umsetzung in Abwesenheit von Lösemitteln und/oder ohne jedwedes Lösemittel durchgeführt wird; und/oder
wobei die Umsetzung autokatalytisch oder in Gegenwart eines Katalysators, insbesondere einer mineralischen Säure, vorzugsweise autokatalytisch, durchgeführt wird; und/oder
wobei die Umsetzung in Gegenwart eines Katalysators, insbesondere einer mineralischen Säure, durchgeführt wird; insbesondere wobei der Katalysator und/oder die mineralische Säure ausgewählt wird/werden aus Schwefelsäure, Halogenwasserstoffsäuren, Phosphorsäuren und deren Mischungen.

3. Verfahren nach Anspruch 1 oder Anspruch 2,
wobei die Verbindung der allgemeinen Formel (I), bezogen auf die Hydroxylgruppen des Polyols (II), insbesondere Polyglycerins, in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird; und/oder
wobei die Verbindung der allgemeinen Formel (I) und das Polyol (II), insbesondere Polyglycerin, in einem Molverhältnis von Verbindung der allgemeinen Formel (I) / Polyol (II) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

4. Verfahren nach einem der vorangehenden Ansprüche,
wobei das Polyol (II) mindestens drei Hydroxylgruppen (OH-Gruppen) enthält; und/oder wobei das Polyol (II) der allgemeinen Formel (IIa)
(HO)ₘ - (X) - (OH)ₙ (IIa)
entspricht, wobei in der allgemeinen Formel (IIa)
• X einen organischen Rest, insbesondere einen 4 bis 20 Kohlenstoffatome enthaltenden und gegebenenfalls 1 bis 9 Sauerstoffatome enthaltenden, vorzugsweise gesättigten organischen Rest darstellt, vorzugsweise ausgewählt aus einem Alkylrest oder einem (Poly-)Alkylether-Rest, insbesondere (Poly-)Alkylenglykolrest, besonders bevorzugt ausgewählt aus einem C₄-C₂₀-Alkylrest oder einem C₄-C₂₀-(Poly-)Alkylether-Rest, insbesondere C₄-C₂₀-(Poly-)Alkylenglykolrest; und
• die Variablen m und n, jeweils unabhängig voneinander, eine ganze Zahl von 1 bis 10 darstellen;
insbesondere wobei die Hydroxylgruppen des Polyols (II) in beliebigen Positionen des Rests X befindlich sind, vorzugsweise wobei mindestens eine Hydroxylgruppe endständig ist und/oder eine primäre Hydroxylgruppe ist.

5. Verfahren nach einem der vorangehenden Ansprüche,
wobei das Polyol (II) ausgewählt ist aus Polyetherpolyolen und Alkanpolyolen sowie deren Kombinationen, insbesondere C₄-C₂₀-Polyetherpolyolen und C₄-C₂₀-Alkanpolyolen, vorzugsweise C₄-C₂₀-Polyetherpolyolen und C₄-C₂₀-Alkandiolen, besonders bevorzugt Polyetherpolyolen, ganz besonders bevorzugt C₄-C₂₀-Polyetherpolyolen; und/oder
wobei das Polyol (II) ausgewählt ist aus Polyetherpolyolen, insbesondere C₄-C₂₀-Polyetherpolyolen, vorzugsweise Polyglycerinen der allgemeinen Formel (IIb)
HO - CH₂ - CH(OH) - CH₂ - [O - CH₂ - CH(OH) - CH₂]ₚ - OH (IIb)
wobei in der allgemeinen Formel (IIb) die Variable p eine ganze Zahl von 1 bis 4, insbesondere 1 oder 2, vorzugsweise 1, darstellt; und/oder
wobei das Polyol (II) ein Diglycerin der Formel (IIc)
HO - CH₂ - CH(OH) - CH₂ - O - CH₂ - CH(OH) - CH₂ - OH (IIc)
ist; und/oder
wobei das Polyol (II) ausgewählt ist aus Alkandiolen, insbesondere C₄-C₂₀-Alkandiolen, vorzugsweise linearen oder verzweigten Alkandiolen, bevorzugt linearen oder verzweigten C₄-C₂₀-Alkandiolen, besonders bevorzugt linearen C₄-C₂₀-Alkandiolen, ganz besonders bevorzugt linearen C₄-C₂₀-Alkandiolen mit mindestens einer endständigen und/oder primären Hydroxylgruppe, noch mehr bevorzugt Pentandiol, insbesondere 1,2-Pentandiol.

6. Verfahren nach einem der vorangehenden Ansprüche,
wobei im Reaktionsprodukt (III) nach erfolgter Umsetzung noch vorhandene Hydroxylgruppen zumindest teilweise, vorzugsweise vollständig, funktionalisiert, insbesondere verestert werden; und/oder
wobei sich der Umsetzung eine teilweise, insbesondere vollständige Funktionalisierung, insbesondere Veresterung, noch vorhandener Hydroxylgruppen anschließt; und/oder wobei die Funktionalisierung, insbesondere Veresterung, der Hydroxylgruppen durch Reaktion mit einem Carbonsäureanhydrid, insbesondere C₂-C₃₀-Carbonsäureanhydrid, bevorzugt C₂-C₁₀-Carbonsäureanhydrid, vorzugsweise C₇-Carbonsäureanhydrid, durchgeführt wird, insbesondere wobei das C₂-C₃₀-Carbonsäureanhydrid, bevorzugt C₂-C₁₀-Carbonsäureanhydrid, vorzugsweise C₇-Carbonsäureanhydrid, ein lineares (geradkettiges) oder verzweigtes, gesättigtes oder ein- oder mehrfach ungesättigtes C₂-C₃₀-Carbonsäureanhydrid, bevorzugt C₂-C₁₀-Carbonsäureanhydrid, vorzugsweise C₇-Carbonsäureanhydrid, ist; und/oder
wobei die Funktionalisierung, insbesondere Veresterung, der Hydroxylgruppen durch Reaktion mit einem Carbonsäureanhydrid, insbesondere mit einer Verbindung der allgemeinen Formel (I), wie zuvor definiert, durchgeführt wird.

7. Polyolester, insbesondere Polyglycerinester, von gegebenenfalls acylierter 3-Hydroxybuttersäure,
wobei der Polyolester der allgemeinen Formel (IIIb)
R²O - CH₂ - CH(OR²) - CH₂ - [O- CH₂ - CH(OR²)- CH₂]ₚ- OR² (IIIb)
entspricht,
wobei in der allgemeinen Formel (IIIb)
• die Variable p eine ganze Zahl von 1 bis 4, insbesondere 1 oder 2, vorzugsweise 1, darstellt,
• R², unabhängig voneinander, darstellt: Wasserstoff, einen Rest CH₃ - CH(OH) - CH₂ - C(O) - oder einen Rest CH₃ - CH(OR¹)- CH₂ - C(O) - mit R¹ = Acylgruppe, ausgewählt aus - C(O) - CH₃ (Acetylgruppe) oder - C(O) - C₂H₅ (Propionylgruppe), bevorzugt - C(O) - CH₃ (Acetylgruppe), jedoch mit der Maßgabe, dass mindestens ein Rest R², insbesondere mindestens zwei Reste R², keinen Wasserstoff darstellt, und/oder mit der Maßgabe, dass mindestens ein Rest R², insbesondere mindestens zwei Reste R², einen Rest CH₃ - CH(OH) - CH₂ - C(O) - oder einen Rest CH₃ - CH(OR¹)- CH₂ - C(O) - mit R¹ wie zuvor definiert darstellt.

8. Polyolester nach Anspruch 7,
wobei in der allgemeinen Formel (IIIb) R², unabhängig voneinander, Wasserstoff oder einen Rest CH₃ - CH(OH) - CH₂ - C(O) - darstellt, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OH) - CH₂ - C(O) - darstellt; oder aber wobei in der allgemeinen Formel (IIIb) R², unabhängig voneinander, Wasserstoff oder einen Rest CH₃ - CH(OR¹)- CH₂ - C(O) - mit R¹ wie zuvor definiert darstellt, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OR¹)- CH₂ - C(O) - mit R¹ wie zuvor definiert darstellt; oder aber
wobei in der allgemeinen Formel (IIIb) R², unabhängig voneinander, darstellt: einen Rest CH₃ - CH(OH) - CH₂ - C(O) - oder einen Rest CH₃ - CH(OR¹)- CH₂ - C(O) - mit R¹ wie zuvor definiert.

9. Polyolester, insbesondere Polyglycerinester, von gegebenenfalls acylierter 3-Hydroxybuttersäure, nach Anspruch 7 oder Anspruch 8,
wobei der Polyolester der allgemeinen Formel (IIIc),
R²O - CH₂ - CH(OR²)- CH₂ - O - CH₂ - CH(OR²)- CH₂ - OR² (IIIc)
entspricht, wobei in der allgemeinen Formel (IIIc) R², unabhängig voneinander, darstellt: Wasserstoff, einen Rest CH₃ - CH(OH) - CH₂ - C(O) - oder einen Rest CH₃ - CH(OR¹)- CH₂ - C(O) - mit R¹ = Acylgruppe, ausgewählt aus - C(O) - CH₃ (Acetylgruppe) oder - C(O) - C₂H₅ (Propionylgruppe), bevorzugt - C(O) - CH₃ (Acetylgruppe), jedoch mit der Maßgabe, dass mindestens ein Rest R², insbesondere mindestens zwei Reste R², keinen Wasserstoff darstellt, und/oder mit der Maßgabe, dass mindestens ein Rest R², insbesondere mindestens zwei Reste R², einen Rest CH₃ - CH(OH) - CH₂ - C(O) - oder einen Rest CH₃ - CH(OR¹)- CH₂ - C(O) - mit R¹ wie zuvor definiert darstellt.

10. Polyolester nach Anspruch 9,
wobei in der allgemeinen Formel (IIIc) R², unabhängig voneinander, Wasserstoff oder einen Rest CH₃ - CH(OH) - CH₂ - C(O) - darstellt, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OH) - CH₂ - C(O) - darstellt; oder aber wobei in der allgemeinen Formel (IIIc) R², unabhängig voneinander, Wasserstoff oder einen Rest CH₃ - CH(OR¹)- CH₂ - C(O) - mit R¹ wie zuvor definiert darstellt, jedoch mit der Maßgabe, dass mindestens ein Rest R², insbesondere mindestens zwei Reste R², keinen Wasserstoff darstellt und/oder mit der Maßgabe, dass mindestens ein Rest R², insbesondere mindestens zwei Reste R², einen Rest CH₃ - CH(OR¹)- CH₂ - C(O) - mit R¹ wie zuvor definiert darstellt; oder aber
wobei in der allgemeinen Formel (IIIc) R², unabhängig voneinander, darstellt: einen Rest CH₃ - CH(OH) - CH₂ - C(O) - oder einen Rest CH₃ - CH(OR¹)- CH₂ - C(O) - mit R¹ wie zuvor definiert.

11. Gemisch, umfassend mindestens zwei, insbesondere mindestens drei, voneinander verschiedene Polyolester, insbesondere Polyglycerinester, von gegebenenfalls acylierter 3-Hydroxybuttersäure, gemäß einem der Ansprüche 7 bis 10.

12. Pharmazeutische Zusammensetzung, insbesondere Arzneimittel oder Medikament, umfassend einen Polyolester gemäß einem der Ansprüche 7 bis 10 und/oder ein Gemisch gemäß Anspruch 11.

13. Pharmazeutische Zusammensetzung nach Anspruch 12 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

14. Polyolester gemäß einem der Ansprüche 7 bis 10 und/oder Gemisch gemäß Anspruch 11 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

15. Verwendung eines Polyolesters gemäß einem der Ansprüche 7 bis 10 und/oder eines Gemischs gemäß Anspruch 11 zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

16. Verwendung eines Polyolesters gemäß einem der Ansprüche 7 bis 10 und/oder eines Gemischs gemäß Anspruch 11 zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung.

17. Nahrungsmittel- und/oder Lebensmittelerzeugnis, umfassend einen Polyolester gemäß einem der Ansprüche 7 bis 10 und/oder ein Gemisch gemäß Anspruch 11.

## Claims

1. Method for the preparation of polyol esters, in particular polyglycerol esters, from optionally acylated 3-hydroxybutyric acid (beta-hydroxybutyric acid, BHB or 3-BHB),
wherein at least one compound of the general formula (I)
CH₃ - CH(OR¹) - CH₂ - C(O) - O - C(O) - CH₂ - CH(OR¹) - CH₃ (I)
wherein in the general formula (I) the radical R¹is an acyl group selected from - C(O) - CH₃ (acetyl group) or - C(O) - C₂H₅ (propionyl group), preferably - C(O) - CH₃ (acetyl group), is reacted with at least one polyol (II) containing at least two hydroxyl groups (OH groups), in particular polyglycerol, optionally followed by hydrolysis of the acyl groups,
so that one or more polyol esters, in particular polyglycerol esters, of optionally acylated 3-hydroxybutyric acid are obtained as reaction product (III).

2. Method according to claim 1,
wherein the reaction is carried out in the absence of solvents and/or without any solvent; and/or
wherein the reaction is carried out autocatalytically or in the presence of a catalyst, in particular a mineral acid, preferably autocatalytically; and/or
wherein the reaction is carried out in the presence of a catalyst, in particular a mineral acid; in particular wherein the catalyst and/or the mineral acid is/are selected from sulfuric acid, hydrohalic acids, phosphoric acids and mixtures thereof.

3. Method according to claim 1 or claim 2,
wherein the compound of the general formula (I), based on the hydroxyl groups of the polyol (II), in particular polyglycerol, is used in molar amounts in a range from an equimolar amount up to a molar excess of 200 mol-%, in particular in a range from an equimolar amount up to a molar excess of 150 mol-%, preferably in a range from an equimolar amount up to a molar excess of 100 mol-%; and/or
wherein the compound of the general formula (I) and the polyol (II), in particular polyglycerol, are used in a molar ratio of compound of the general formula (I) / polyol (II) in a range from 1 : 1 to 10 : 1, in particular in a range from 2 : 1 to 8 : 1, preferably in a range from 3 : 1 to 6 : 1.

4. Method according to any one of the preceding claims,
wherein the polyol (II) contains at least three hydroxyl groups (OH groups); and/or wherein the polyol (II) of the general formula (IIa)
(HO)ₘ- (X) - (OH)ₙ (IIa)
wherein in the general formula (IIa)
• X is an organic radical, in particular an organic radical containing 4 to 20 carbon atoms and optionally containing 1 to 9 oxygen atoms, preferably a saturated organic radical, preferably selected from an alkyl radical or a (poly)alkyl ether radical, in particular a (poly)alkylene glycol radical, especially preferably selected from a C₄-C₂₀-alkyl radical or a C₄-C₂₀-(poly)alkylene ether radical, in particular a C₄-C₂₀-(poly)alkylene glycol radical; and
• the variables m and n, independently of each other, represent an integer from 1 to 10; in particular wherein the hydroxyl groups of the polyol (II) are located in any positions of the residue X, preferably wherein at least one hydroxyl group is terminal and/or is a primary hydroxyl group.

5. Method according to any one of the preceding claims,
wherein the polyol (II) is selected from polyether polyols and alkane polyols and combinations thereof, in particular C₄-C₂₀-polyether polyols and C₄-C₂₀-alkane polyols, preferably C₄-C₂₀-polyetherpolyols and C₄-C₂₀-alkanediols, particularly preferably polyetherpolyols, most preferably C₄-C₂₀-polyetherpolyols; and/or wherein the polyol (II) is selected from polyether polyols, in particular C₄-C₂₀-polyether polyols, preferably polyglycerols of the general formula (IIb)
HO - CH₂ - CH(OH) - CH₂ - [O - CH₂ - CH(OH) - CH₂]ₚ- OH (IIb)
wherein in the general formula (IIb) the variable p represents an integer from 1 to 4, in particular 1 or 2, preferably 1; and/or
wherein the polyol (II) is a diglycerol of formula (IIc)
HO - CH₂ - CH(OH) -CH₂-O - CH₂ - CH(OH) - CH₂ - OH (IIc)
; and/or
wherein the polyol (II) is selected from alkanediols, in particular C₄-C₂₀-alkanediols, preferably linear or branched alkanediols, preferably linear or branched C₄-C₂₀-alkanediols, particularly preferably linear C₄-C₂₀-alkanediols, very particularly preferably linear C₄-C₂₀-alkanediols with at least one terminal and/or primary hydroxyl group, even more preferably pentanediol, in particular 1,2-pentanediol.

6. Method according to any one of the preceding claims,
wherein hydroxyl groups still present in the reaction product (III) after the reaction has taken place are at least partially, preferably completely, functionalized, in particular esterified; and/or
wherein the reaction is followed by a partial, in particular complete functionalization, in particular esterification, of hydroxyl groups still present; and/or
wherein the functionalization, in particular esterification, of the hydroxyl groups is carried out by reaction with a carboxylic acid anhydride, in particular C₂-C₃₀-carboxylic acid anhydride, preferably C₂-C₁₀-carboxylic acid anhydride, preferably C₇-carboxylic acid anhydride, in particular wherein the C₂-C₃₀-carboxylic acid anhydride, preferably C₂-C₁₀-carboxylic acid anhydride, preferably C₇-carboxylic acid anhydride, is a linear (straight-chain) or branched, saturated or mono- or polyunsaturated C₂-C₃₀-carboxylic acid anhydride, preferably C₂-C₁₀-carboxylic acid anhydride, preferably C₇-carboxylic acid anhydride; and/or
wherein the functionalization, in particular esterification, of the hydroxyl groups is carried out by reaction with a carboxylic acid anhydride, in particular with a compound of the general formula (I) as defined above.

7. Polyol esters, in particular polyglycerol esters, of optionally acylated 3-hydroxybutyric acid, wherein the polyol ester corresponds to the general formula (IIIb)
R²O - CH₂ - CH(OR²)- CH₂ - [O - CH₂ - CH(OR²)- CH₂]ₚ- OR² (IIIb),
wherein in the general formula (IIIb)
• the variable p represents an integer from 1 to 4, in particular 1 or 2, preferably 1,
• R², independently of one another: hydrogen, a radical CH₃ - CH(OH) - CH₂ - C(O) - or a radical CH₃ - CH(OR¹) - CH₂ - C(O) - with R¹ = acyl group, selected from -C(O)-CH₃ (acetyl group) or -C(O)-C₂H₅ (propionyl group), preferably - C(O) - CH₃ (acetyl group), but with the proviso that at least one radical R², in particular at least two radicals R², is not hydrogen, and/or with the proviso that at least one radical R², in particular at least two radicals R², is a radical CH₃ - CH(OH) - CH₂ - C(O) - or a radical CH₃ - CH(OR¹)- CH₂ - C(O) - with R¹as defined above.

8. Polyol ester according to claim 7,
wherein, in the general formula (IIIb), R², independently of one another, is hydrogen or a radical CH₃ - CH(OH) - CH₂ - C(O) -, but with the proviso that at least one radical R², in particular at least two radicals R², is not hydrogen and/or with the proviso that at least one radical R², in particular at least two radicals R², is a radical CH₃ - CH(OH) - CH₂ - C(O) ; or else
wherein, in the general formula (IIIb), R², independently of one another, is hydrogen or a radical CH₃ - CH(OR¹)- CH₂ - C(O) - with R¹as defined above, but with the proviso that at least one radical R², in particular at least two radicals R², is not hydrogen and/or with the proviso that at least one radical R², in particular at least two radicals R², is a radical CH₃ - CH(OR¹)- CH₂ - C(O) - with R¹as defined above; or else
wherein, in the general formula (IIIb), R², independently of one another, represents: a radical CH₃ - CH(OH) - CH₂ - C(O) - or a radical CH₃ - CH(OR¹)- CH₂ - C(O) - with R¹as defined above.

9. Polyol esters, in particular polyglycerol esters, of optionally acylated 3-hydroxybutyric acid, according to claim 7 or claim 8,
wherein the polyol ester corresponds to the general formula (IIIc),
R²O - CH₂ - CH(OR²)- CH₂ - O - CH₂ - CH(OR²)- CH₂ - OR² (IIIc)
wherein in the general formula (IIIc) R²represents, independently of one another: hydrogen, a radical CH₃ - CH(OH) - CH₂ - C(O) - or a radical CH₃ - CH(OR¹)- CH₂ - C(O) - with R¹ = acyl group, selected from - C(O) - CH₃ (acetyl group) or - C(O) - C₂H₅ (propionyl group), preferably - C(O) - CH₃ (acetyl group), but with the proviso that at least one radical R², in particular at least two radicals R², is not hydrogen, and/or with the proviso that at least one radical R², in particular at least two radicals R², is a radical CH₃ - CH(OH) - CH₂ - C(O) - or a radical CH₃ - CH(OR¹)- CH₂ - C(O) - with R¹as defined above.

10. Polyol ester according to claim 9,
wherein, in the general formula (IIIc), R², independently of one another, represents hydrogen or a radical CH₃ - CH(OH) - CH₂ - C(O) -, but with the proviso that at least one radical R², in particular at least two radicals R², does not represent hydrogen and/or with the proviso that at least one radical R², in particular at least two radicals R², represents a radical CH₃ - CH(OH) - CH₂ - C(O) -; or else
wherein, in the general formula (IIIc), R², independently of one another, is hydrogen or a radical CH₃ - CH(OR¹)- CH₂ - C(O) - with R¹ as defined above, but with the proviso that at least one radical R², in particular at least two radicals R², is not hydrogen and/or with the proviso that at least one radical R², in particular at least two radicals R², is a radical CH₃ - CH(OR¹)- CH₂ - C(O) - with R¹as defined above; or else
wherein, in the general formula (IIIc), R², independently of one another, represents: a radical CH₃ - CH(OH) - CH₂ - C(O) - or a radical CH₃ - CH(OR¹)- CH₂ - C(O) - with R¹as defined above.

11. Mixture comprising at least two, in particular at least three, mutually different polyol esters, in particular polyglycerol esters, of optionally acylated 3-hydroxybutyric acid, according to any one of claims 7 to 10.

12. Pharmaceutical composition, in particular a drug or medicament, comprising a polyol ester according to any one of claims 7 to 10 and/or a mixture according to claim 11.

13. Pharmaceutical composition according to claim 12 for use in the prophylactic and/or therapeutic treatment of diseases of the human or animal body, in particular diseases associated with a disorder of energy metabolism, in particular keto-body metabolism, such as in particular craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, lipometabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, in particular ulcerative colitis and Crohn's disease, lyosomal storage diseases such as sphingolipidoses, in particular Niemann-Pick disease, diabetes mellitus and the effects or side effects of chemotherapy.

14. Polyol ester according to any one of claims 7 to 10 and/or mixture according to claim 11 for use in the prophylactic and/or therapeutic treatment of diseases of the human or animal body, in particular diseases associated with a disorder of energy metabolism, in particular keto-body metabolism, such as in particular craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, lipometabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, in particular ulcerative colitis and Crohn's disease, lyosomal storage diseases such as sphingolipidoses, in particular Niemann-Pick disease, diabetes mellitus and the effects or side effects of chemotherapy.

15. Use of a polyol ester according to any one of claims 7 to 10 and/or a mixture according to claim 11 for the manufacture of a medicament for the prophylactic and/or therapeutic treatment of diseases of the human or animal body, in particular diseases associated with a disorder of energy metabolism, in particular keto-body metabolism, such as in particular craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, lipid metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, in particular ulcerative colitis and Crohn's disease, lyosomal storage diseases such as sphingolipidoses, in particular Niemann-Pick disease, diabetes mellitus and the effects or side effects of chemotherapy.

16. Use of a polyol ester according to any one of claims 7 to 10 and/or a mixture according to claim 11 for the manufacture of a medicament for the prophylactic and/or therapeutic treatment or use of/for catabolic metabolic situations, such as starvation, diets or low-carbohydrate diets.

17. Food and/or food product comprising a polyol ester according to any one of claims 7 to 10 and/or a mixture according to claim 11.

## Revendications

1. Procédé de préparation d'esters de polyols, en particulier d'esters de polyglycérol, d'acide 3-hydroxybutyrique éventuellement acylé (acide bêta-hydroxybutyrique, BHB ou 3-BHB),
où au moins un composé de formule générale (I)
CH₃ - CH(OR¹)- CH₂ - C(O) - O - C(O) - CH₂ - CH(OR¹)- CH₃ (I)
où, dans la formule générale (I), le radical R¹ est un groupe acyle choisi parmi - C(O) - CH₃ (groupe acétyle) ou - C(O) - C₂H₅ (groupe propionyle), de préférence - C(O) - CH₃ (groupe acétyle),
est mis à réagir avec au moins un polyol (II) contenant au moins deux groupes hydroxyle (groupes OH), en particulier du polyglycérol, éventuellement suivi d'une hydrolyse des groupes acyle,
de sorte que l'on obtient comme produit de réaction (III) un ou plusieurs esters de polyol, en particulier des esters de polyglycérol, d'acide 3-hydroxybutyrique éventuellement acylé.

2. Procédé selon la revendication 1,
la réaction étant effectuée en l'absence de solvants et/ou sans aucun solvant; et/ou la réaction étant effectuée de manière autocatalytique ou en présence d'un catalyseur, en particulier un acide minéral, de préférence de manière autocatalytique; et/ou
la réaction étant effectuée en présence d'un catalyseur, en particulier d'un acide minéral; en particulier où le catalyseur et/ou l'acide minéral est/sont choisi(s) parmi l'acide sulfurique, les acides halogénhydriques, les acides phosphoriques et leurs mélanges.

3. Procédé selon la revendication 1 ou la revendication 2,
le composé de formule générale (I) étant utilisé, par rapport aux groupes hydroxyle du polyol (II), en particulier du polyglycérol, en quantités molaires dans une plage allant d'une quantité équimolaire jusqu'à un excès molaire de 200 % en moles, en particulier dans une plage allant d'une quantité équimolaire jusqu'à un excès molaire de 150 % en moles, de préférence dans une plage allant d'une quantité équimolaire jusqu'à un excès molaire de 100 % en moles; et/ou le composé de formule générale (I) et le polyol (II), en particulier le polyglycérol, étant utilisés dans un rapport molaire composé de formule générale (I)/polyol (II) dans une plage de 1 : 01 à 10 : 1, en particulier dans une plage de 2 : 1 à 8 : 1, de préférence dans une plage de 3 : 1 à 6 : 1.

4. Procédé selon l'une quelconque des revendications précédentes,
le polyol (II) contenant au moins trois groupes hydroxyle (groupes OH); et/ou où le polyol (II) correspond à la formule générale (IIa)
(HO)ₘ- (X) - (OH)ₙ (IIa)
où, dans la formule générale (IIa),
• X représente un radical organique, en particulier un radical organique contenant de 4 à 20 atomes de carbone et contenant éventuellement de 1 à 9 atomes d'oxygène, de préférence saturé, choisi de préférence parmi un radical alkyle ou un radical (poly)alkyléther, en particulier un radical (poly)alkylène glycol, de préférence encore choisi parmi un radical C₄-C₂₀-alkyle ou un radical C₄-C₍₂₀₎-(poly)alkyléther, en particulier un radical C₄-C₍₂₀₎-(poly)alkylène glycol; et
• les variables m et n représentent chacune, indépendamment l'une de l'autre, un nombre entier de 1 à 10;
en particulier où les groupes hydroxyle du polyol (II) sont situés dans des positions quelconques du résidu X, de préférence où au moins un groupe hydroxyle est terminal et/ou est un groupe hydroxyle primaire.

5. Procédé selon l'une quelconque des revendications précédentes,
le polyol (II) étant choisi parmi les polyétherpolyols et les alcanpolyols ainsi que leurs combinaisons, en particulier les C₄-C₂₀-polyétherpolyols et les C₄-C₂₀-alcanpolyols, de préférence des C₄-C₂₀-polyétherpolyols et des C₄-C₂₀-alcanediols, de préférence des polyétherpolyols, de préférence encore des C₄-C₂₀-polyétherpolyols; et/ou
le polyol (II) étant choisi parmi les polyétherpolyols, en particulier les polyétherpolyols C₄-C₂₀, de préférence les polyglycérols de formule générale (IIb)
HO - CH₂ - CH(OH) - CH₂ - [O - CH₂ - CH(OH) - CH₂]ₚ- OH (IIb)
où, dans la formule générale (IIb), la variable p représente un nombre entier de 1 à 4, en particulier 1 ou 2, de préférence 1; et/ou
le polyol (II) étant un diglycérol de formule (IIc)
HO - CH₂ - CH(OH) - CH₂ - O - CH₂ - CH(OH) - CH₂ - OH (IIc)
et/ou
le polyol (II) étant choisi parmi des alcanediols, en particulier des alcanediols C₄-C₂₀, de préférence des alcanediols linéaires ou ramifiés, de préférence des alcanediols C₄-C₂₀ linéaires ou ramifiés, de préférence des C₄-C₂₀-alcanediols linéaires, de manière tout à fait préférée des C₄-C₂₀-alcanediols linéaires avec au moins un groupe hydroxyle terminal et/ou primaire, de manière encore plus préférée le pentanediol, en particulier le 1,2-pentanediol.

6. Procédé selon l'une quelconque des revendications précédentes,
les groupes hydroxyle encore présents dans le produit de réaction (III) après la réaction étant au moins partiellement, de préférence complètement, fonctionnalisés, en particulier estérifiés; et/ou
la réaction étant suivie d'une fonctionnalisation partielle, en particulier complète, en particulier d'une estérification, des groupes hydroxyle encore présents; et/ou
la fonctionnalisation, en particulier l'estérification, des groupes hydroxyle étant réalisée par réaction avec un anhydride d'acide carboxylique, en particulier l'anhydride d'acide carboxylique C₂-C₃₀, de préférence l'anhydride d'acide carboxylique C₂-C₁₀, de préférence l'anhydride d'acide carboxylique C₇, en particulier où l'anhydride d'acide carboxylique C₂-C₃₀, de préférence l'anhydride d'acide C₂-C₁₀-carboxylique, de préférence l'anhydride d'acide C₇-carboxylique, est un anhydride d'acide C₂-C₃₀-carboxylique linéaire (à chaîne droite) ou ramifié, saturé ou mono- ou polyinsaturé, de préférence l'anhydride d'acide C₂-C₁₀-carboxylique, de préférence l'anhydride d'acide C₇-carboxylique; et/ou
la fonctionnalisation, en particulier l'estérification, des groupes hydroxyle étant réalisée par réaction avec un anhydride d'acide carboxylique, en particulier avec un composé de formule générale (I) telle que définie précédemment.

7. Ester de polyols, en particulier ester de polyglycérol, de l'acide 3-hydroxybutyrique éventuellement acylé,
où l'ester de polyol correspond à la formule générale (IIIb)
R²O - CH₂ - CH(OR²)- CH₂ - [O - CH₂ - CH(OR²)- CH₂]ₚ- OR² (IIIb)
où, dans la formule générale (IIIb)
• la variable p représente un nombre entier de 1 à 4, en particulier 1 ou 2, de préférence 1,
• R², indépendamment l'un de l'autre: l'hydrogène, un radical CH₃ - CH(OH) - CH₂ - C(O) - ou un radical CH₃ - CH(OR¹)- CH₂ - C(O) - avec R¹ = groupe acyle, choisi parmi - C(O) - CH₃ (groupe acétyle) ou - C(O) - C₂H₅ (groupe propionyle), de préférence - C(O) - CH₃ (groupe acétyle), à condition toutefois qu'au moins un radical R², en particulier au moins deux radicaux R², ne représente pas un atome d'hydrogène, et/ou à condition qu'au moins un radical R², en particulier au moins deux radicaux R², représente un radical CH₃ - CH(OH) - CH₂ - C(O) - ou un radical CH₃ - CH(OR¹)- CH₂ - C(O) - avec R¹ tel que défini précédemment.

8. Ester de polyol selon la revendication 7,
où, dans la formule générale (IIIb), R², indépendamment l'un de l'autre, représente l'hydrogène ou un radical CH₃ - CH(OH) - CH₂ - C(O) -, à condition toutefois qu'au moins un radical R², en particulier au moins deux radicaux R², ne représente pas l'hydrogène et/ou à condition qu'au moins un radical R², en particulier au moins deux radicaux R², représente un radical CH₃- CH(OH) - CH₂ - C(O) -; ou bien
où, dans la formule générale (IIIb), R², indépendamment l'un de l'autre, représente un atome d'hydrogène ou un radical CH₃ - CH(OR¹)- CH₂ - C(O) - avec R¹tel que défini précédemment, à condition toutefois qu'au moins un radical R², en particulier au moins deux radicaux R², ne représente pas un hydrogène et/ou à condition qu'au moins un radical R², en particulier au moins deux radicaux R², représente un radical CH₃ - CH(OR¹)- CH₂ - C(O) - avec R¹ tel que défini précédemment; ou bien
où, dans la formule générale (IIIb), R² représente indépendamment: un radical CH₃ - CH(OH) - CH₂ - C(O) - ou un radical CH₃ - CH(OR¹)- CH₂ - C(O) - avec R¹ tel que défini précédemment.

9. Ester de polyols, en particulier ester de polyglycérol, de l'acide 3-hydroxy-butyrique éventuellement acylé, selon la revendication 7 ou la revendication 8,
où l'ester de polyol correspond à la formule générale (IIIc),
R²O - CH₂ - CH(OR²)- CH₂ - O - CH₂ - CH(OR²)- CH₂ - OR² (IIIc)
où, dans la formule générale (IIIc), R², indépendamment l'un de l'autre, représente: l'hydrogène, un radical CH₃ - CH(OH) - CH₂ - C(O) - ou un radical CH₃ - CH(OR¹)- CH₂ - C(O) - avec R¹ = groupe acyle, choisi parmi - C(O) - CH₃ (groupe acétyle) ou - C(O) - C₂H₅ (groupe propionyle), de préférence - C(O) - CH₃ (groupe acétyle), à condition toutefois qu'au moins un radical R², en particulier au moins deux radicaux R², ne représente pas un hydrogène, et/ou à condition qu'au moins un radical R², en particulier au moins deux radicaux R², représente un radical CH₃ - CH(OH) - CH₂ - C(O) - ou un radical CH₃ - CH(OR¹)- CH₂ - C(O) - avec R¹ tel que défini précédemment.

10. Ester de polyol selon la revendication 9,
où, dans la formule générale (IIIc), R², indépendamment l'un de l'autre, représente l'hydrogène ou un radical CH₃ - CH(OH) - CH₂ - C(O) -, à condition toutefois qu'au moins un radical R², en particulier au moins deux radicaux R², ne représente pas l'hydrogène et/ou à condition qu'au moins un radical R², en particulier au moins deux radicaux R², représente un radical CH₃ - CH(OH) - CH₂ - C(O) -; ou bien
où, dans la formule générale (IIIc), R², indépendamment l'un de l'autre, représente un atome d'hydrogène ou un radical CH₃ - CH(OR¹)- CH₂ - C(O) - avec R¹tel que défini précédemment, à condition toutefois qu'au moins un radical R², en particulier au moins deux radicaux R², ne représente pas un hydrogène et/ou à condition qu'au moins un radical R², en particulier au moins deux radicaux R², représente un radical CH₃ - CH(OR¹)- CH₂ - C(O) - avec R¹ tel que défini précédemment; ou bien
où, dans la formule générale (IIIc), R² représente, indépendamment: un radical CH₃ - CH(OH) - CH₂ - C(O) - ou un radical CH₃ - CH(OR¹)- CH₂ - C(O) - avec R¹ tel que défini précédemment.

11. Mélange comprenant au moins deux, en particulier au moins trois, esters de polyol différents les uns des autres, en particulier esters de polyglycérol, d'acide 3-hydroxybutyrique éventuellement acylé, selon l'une quelconque des revendications 7 à 10.

12. Composition pharmaceutique, en particulier médicament ou médicament, comprenant un ester de polyol selon l'une quelconque des revendications 7 à 10 et/ou un mélange selon la revendication 11.

13. Composition pharmaceutique selon la revendication 12 pour son utilisation dans le traitement prophylactique et/ou thérapeutique de maladies du corps humain ou animal, en particulier de maladies liées à un trouble du métabolisme énergétique, en particulier du métabolisme des corps cétoniques, comme notamment les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

14. Ester de polyols selon l'une quelconque des revendications 7 à 10 et/ou mélange selon la revendication 11, pour leur utilisation dans le traitement prophylactique et/ou thérapeutique de maladies du corps humain ou animal, en particulier de maladies liées à un trouble du métabolisme énergétique, en particulier le métabolisme des corps cétoniques, comme en particulier les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules **T,** les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

15. Utilisation d'un ester de polyol selon l'une des revendications 7 à 10 et/ou d'un mélange selon la revendication 11 pour la fabrication d'un médicament destiné au traitement prophylactique et/ou thérapeutique de maladies du corps humain ou animal, en particulier de maladies liées à un trouble du métabolisme énergétique, en particulier le métabolisme des corps cétoniques, comme en particulier les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, sclérose en plaques et sclérose latérale amyotrophique, maladies du métabolisme lipidique telles que le déficit en transporteur de glucose (déficit GLUT1), VL-FAOD et mitochondriopathies telles que le déficit en thiolase mitochondriale, la chorée de Huntington, maladies cancéreuses telles que les lymphomes à cellules **T,** astrocytomes et glioblastomes, VIH, maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

16. Utilisation d'un ester de polyol selon l'une quelconque des revendications 7 à 10 et/ou d'un mélange selon la revendication 11 pour la fabrication d'un médicament destiné au traitement prophylactique et/ou thérapeutique ou à l'application de/aux situations métaboliques cataboliques, telles que la faim, les régimes ou les régimes pauvres en hydrates de carbone.

17. Produit alimentaire et/ou nutritionnel comprenant un ester de polyol selon l'une quelconque des revendications 7 à 10 et/ou un mélange selon la revendication 11.
